# EUROPEAN PATENT APPLICATION

(11) **EP 4 209 486 A1**
(43) Date of publication of application: **12.07.2023**
(21) Application number: 22150509.2
(22) Date of filing: 07.01.2022
(51) Int. Cl.: C07D 241/44

(54) **PROCESS FOR THE PREPARATION OF ARYLOXYPHENOXYPROPIONIC ACID DERIVATIVES IN A NON POLAR SOLVENT WITH A TERTIARY AMINE CATALYST**

(71) Applicant: Adama Agan Ltd., 7710001 Ashdod (IL)
(72) Inventor: COHEN, Carmen, 7766095 Ashdod (IL); FRIDMAN, Inna, 7740720 Ashdod (IL)
(74) Representative: Modiano, Gabriella Diana

(57) **Abstract**

A process for the preparation of an aryloxyphenoxypropionic acid derivative compound of formula I 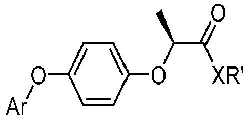 wherein Ar is an aryl group, R' is selected from the group consisting of hydrogen, linear or branched aliphatic, alkoxyalkyl, alicyclic, aralkyl, haloaryl, alkylideneamino-oxyalkyl or heterocyclyl alkyl group, X is selected from NR" or O, and R" is selected from the group consisting of hydrogen, linear or branched aliphatic group, which comprises the reaction in a nonpolar solvent of a phenolpropionic acid derivative compound of formula II 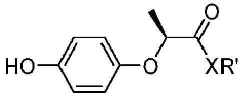 with a haloarene compound of formula Ar-Y when Y is a halogen atom, in the presence of an inorganic or organic base and a catalyst selected from the group of tertiary amines.

## Description

### FIELD OF THE INVENTION

The present invention belongs to the field of agrochemistry. It is directed to a process for preparation of aryloxyphenoxypropionic acid derivatives, that are suitable as herbicides for controlling weeds.

### BACKGROUND

Aryloxyphenoxypropionic acid derivative compounds, also known as FOPs, are a category of herbicides from the family of lipid synthesis inhibitors by inhibition of ACCase. The family of aryloxyphenoxypropionic acid derivative compounds comprises well known herbicides such as for example propaquizafop, quizalofop, clodinafop, cyhalofop, haloxyfop, metamifop, diclofop, fluazifop or fenoxaprop, among other compounds.

The prior art discloses in DE26407030, EP0044497, EP0062905, EP0105494, EP0157225 or WO2004/002925 different methods for preparing aryloxyphenoxypropionic acid derivatives, which are well represented by the following two reaction schemes 1 and 2. 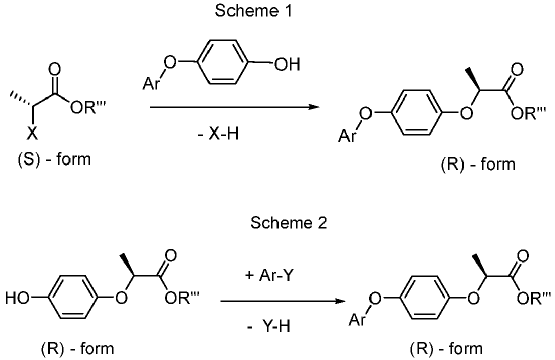 where X is a leaving group such as mesyloxy, tosyloxy, chlorine, or bromine, R"' is an optionally substituted linear or branched aliphatic, alicyclic or heterocyclic group, Ar is an aryl group and Y is a halogen atom.

The main problem of the synthesis of aryloxyphenoxypropionic acid derivatives is the racemization. Due to the presence of a single chiral carbon, aryloxyphenoxypropionic acid derivatives have optical isomers. It is known that (R)-isomers have exceptional herbicidal activities and (S)-isomers have very low herbicidal activities, thus, manufacturing process should be directed to maximizing the (R)-enantiomer content. The removal of (S) enatiomers formed as byproducts, requires of additional difficult and expensive processes to obtain pure (R) enantiomers of aryloxyphenoxypropionic acid derivatives.

The methods according to scheme 1 have the drawback of low enantiomeric purity of the final product, which eventually requires time consuming workup to obtain pure (R) aryloxyphenoxypropionic acid derivatives and recycled tosylate or mesylate derivative, what increased the manufacturing cost.

The methods according to scheme 2 may have different types of solvents. When the solvent is a polar aprotic solvent like for example dimethylformamide, DMSO, N-methyl pyrrolidone, nitriles, dimethylacetamide or ketones, there is a significant disadvantage due to their miscibility with water, what means a difficult and costly solvent recycling. Prior art document EP0105494 discloses other disadvantages for these solvents, like cost of the solvents, low yields, or long reaction times.

Prior art document EP0105494 refers to an approach according to scheme 2, where the solvent is selected among aromatic hydrocarbons, and the reaction is carried out in the presence of a catalyst selected from the group of quaternary ammonium or phosphonium bases, polyalkylene glycols or lower alkyl ethers thereof. Although the yield and purity of the products obtained according to the examples of EP0105494 are higher than 90%, and about 95%, it has been observed a lack of reproducibility.

Therefore, it is the object of the present invention to provide a process for the preparation of aryloxyphenoxypropionic acid derivative herbicides which solves the lack of reproducibility in the reaction between haloarene compounds and phenolpropionic acid derivatives in nonpolar solvents in the presence of a catalyst.

### SUMMARY OF THE INVENTION

A first aspect of the invention is a process for the preparation of an aryloxyphenoxypropionic acid derivative compound of formula I 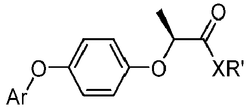 wherein Ar is an aryl group, R' is selected from the group consisting of hydrogen, linear or branched aliphatic, alkoxyalkyl, alicyclic, aralkyl, haloaryl, alkylideneamino-oxyalkyl or heterocyclyl alkyl group, X is selected from NR" or O, and R" is selected from the group consisting of hydrogen, linear or branched aliphatic group, which comprises the reaction in a nonpolar solvent of a phenolpropionic acid derivative compound of formula II 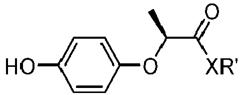 with a haloarene compound of formula Ar-Y when Y is a halogen atom, in the presence of an inorganic or organic base and a catalyst selected from the group of tertiary amines.

### DETAILED DESCRIPTION OF THE INVENTION

### Definitions

Embodiments of the present invention are discussed in detail below. In describing embodiments, specific terminology is employed for the sake of clarity. However, the invention is not intended to be limited to the specific terminology so selected. While specific exemplary embodiments are discussed, it should be understood that this is done for illustration purposes only. A person skilled in the relevant art will recognize that other components and configurations can be used without parting from the spirit and scope of the invention. While a number of embodiments and features are described herein, it is to be understood that the various features of the invention and aspects of embodiments, even if described separately, may be combined unless mutually exclusive or contrary to the specific description. All references cited herein are incorporated by reference as if each had been individually incorporated.

As used herein, the transitional term "comprising" or "that comprises", which is synonymous with "including," or "containing," is inclusive or open-ended and does not exclude additional, un-recited elements or method steps. However, in each recitation of "comprising" herein, it is intended that the term also encompass, as alternative embodiments, the phrases "consisting essentially of" and "consisting of", where "consisting of" excludes any element or step not specified and "consisting essentially of" permits the inclusion of additional un-recited elements or steps that do not materially affect the essential or basic and novel characteristics of the composition or method under consideration.

Prior to setting forth the present subject matter in detail, it may be helpful to provide definitions of certain terms to be used herein. Unless defined otherwise, all technical and scientific terms used herein have the same meaning as is commonly understood by one of skill in the art to which this subject matter pertains.

As used herein, the term "aryl group" refers to an optionally substituted aromatic carbocyclic or heterocyclic group which has between 4 and 10, between 5 and 10, between 5 and 8 carbon atoms, which comprises 1 aromatic ring or 2 fused aromatic rings, wherein the optional substituents are selected from the group consisting of halogen, aliphatic, halo aliphatic, alkoxy, thioalkyl, cyano or nitro groups. In the case of an heterocyclic aryl group, i.e. "heteroaryl group", 1, 2 or 3 of the atoms in the ring or rings of the aryl group, is a different element to carbon, such as nitrogen, oxygen or sulfur.

The term "haloaryl group" refers to an aryl group which has bound at least 1 halogen atom.

The term "aralkyl group" refers to an aryl-substituted alkyl group.

The term "alkoxyalkyl group" refers to an alkoxy-substituted alkyl group, wherein the alkoxy group is an alkyl group bound to oxygen. The alkoxyalkyl group is the radical of an alkyl alkyl ether.

The term "aliphatic group" is used in this invention to cover, for example and not restricted to, the linear or branched alkyl, alkenyl and alkynyl groups.

The term "alkyl group" refers to a saturated, linear or branched group, which has between 1 and 24, between 1 and 16, between 1 and 14, between 1 and 12, 1, 2, 3, 4, 5 or 6 carbon atoms and is bound to the rest of the molecule by a single bond, including, for example and not restricted to, methyl, ethyl, isopropyl, isobutyl, tert-butyl, heptyl, octyl, decyl, dodecyl, hexadecyl, octadecyl, amyl, 2-ethylhexyl, 2-methylbutyl, 5-methylhexyl and similar.

The term "alkenyl group" refers to a linear or branched group, which has between 2 and 24, between 2 and 16, between 2 and 14, between 2 and 12, 2, 3, 4, 5 or 6 carbon atoms, with 1, 2 or 3 carbon-carbon double bonds, conjugated or unconjugated, which is bound to the rest of the molecule by a single bond, including, for example and not restricted to, vinyl, allyl, oleyl, linoleyl and similar groups.

The term "alkynyl group" refers to a linear or branched group, which has between 2 and 24, between 2 and 16, between 2 and 14, between 2 and 12, 2, 3, 4, 5 or 6 carbon atoms, with 1, 2 or 3 carbon-carbon triple bonds, conjugated or unconjugated, which is bound to the rest of the molecule by a single bond, including, for example and not restricted to, the ethynyl group, 1-propinyl, 2-propinyl, 1-butinyl, 2-butinyl, 3-butinyl, pentinyl, such as 1-pentinyl, and similar. The term "alkylidene group" refers to a saturated, linear or branched group, which has between 1 and 24, between 1 and 16, between 1 and 14, between 1 and 12, 1, 2, 3, 4, 5 or 6 carbon atoms and is bound to the rest of the molecule by a double bond, including, for example and not restricted to, methylidene, ethylidene, isopropylidene, isobutylidene, tert-butylidene, heptylidene, octylidene, decylidene, dodecylidene, hexadecylidene and similar.

The term "alycyclic group" is used in this invention to cover, for example and not restricted to, cycloalkyl or cycloalkenyl or cycloalkynyl groups.

The term "cycloalkyl" refers to a saturated mono- or polycyclic aliphatic group which has between 3 and 24, between 3 and 16, between 3 and 14, between 3 and 12, between 3, 4, 5 or 6 carbon atoms and which is bound to the rest of the molecule by a single bond, including, for example and not restricted to, cyclopropyl, cyclobutyl, cyclopentyl, cyclohexyl, cycloheptyl, methyl cyclohexyl, dimethyl cyclohexyl, octahydroindene, decahydronaphthalene, dodecahydrophenalene and similar.

The term "cycloalkenyl" refers to a non-aromatic mono- or polycyclic aliphatic group which has between 5 and 24, between 5 and 16, between 5 and 14, between 5 and 12, 5 or 6 carbon atoms, with 1, 2 or 3 carbon-carbon double bonds, conjugated or unconjugated, and which is bound to the rest of the molecule by a single bond, including, for example and not restricted to, the cyclopent-1-en-1-yl group and similar.

The term "cycloalkynyl" refers to a non-aromatic mono- or polycyclic aliphatic group which has between 8 and 24, between 8 and 16, between 8 and 14, between 8 and 12, 8 or 9 carbon atoms, with 1, 2 or 3 carbon-carbon triple bonds, conjugated or unconjugated, and which is bound to the rest of the molecule by a single bond, including, for example and not restricted to, the cyclooct-2-in-1-yl group and similar.

As used herein, the term "heterocyclic group" refers to a hydrocarbonated ring of 4-10 members, in which one 1, 2 or 3 of the atoms in the ring, is a different element to carbon, such as nitrogen, oxygen or sulfur and can be saturated or unsaturated. For the purposes of this invention, the heterocycle can be a monocyclic or a bicyclic system of condensed rings; and the nitrogen, carbon or sulfur atoms can optionally be oxidized in the radical heterocyclyl; the nitrogen atom can optionally be quaternized; and the radical heterocyclyl can be partially or completely saturated or aromatic. The greatest preference is for the term heterocyclyc to refer to a ring of 5 or 6 members. The radical of an heterocyclic group is also name "heterocyclyl", and for the purposes of this invention the heterocyclyl is bound to the rest of the molecule by a single bond of a carbon atom of the heterocycle.

As used herein, the term "nonpolar solvent" refers to a solvent that is not water miscible, or hardly miscible, and in particular, the term "nonpolar solvent" is used in this invention to cover any solvent which has a dielectric constant of less than 6, measured at 25 ºC.

As used herein, the term "polar solvent" refers to a solvent that is water miscible, and in particular, the term "polar solvent" is used in this invention to cover any solvent which has a dielectric constant of 6 or higher, measured at 25 ºC.

As used herein, the term "reproducibility" relates to the non-variability in terms of yield and optical purity when the same reaction conditions, reactants and amounts are used for different batches.

The term "a" or "an" as used herein includes the singular and the plural, unless specifically stated otherwise. Therefore, the terms "a," "an" or "at least one" can be used interchangeably in this application.

For purposes of better understanding the present teachings and in no way limiting the scope of the teachings, unless otherwise indicated, all numbers expressing quantities, percentages or proportions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques. In this regard, used of the term "about" herein specifically includes ±10% from the indicated values in the range. In addition, the endpoints of all ranges directed to the same component or property herein are inclusive of the endpoints, are independently combinable, and include all intermediate points and ranges. Similarly, the ranges and amounts for each element of the technology described herein can be used together with ranges or amounts for any of the other elements.

### Process for the preparation of aryloxyphenoxypropionic acid derivative compounds

The present disclosure relates to is a process for the preparation of an aryloxyphenoxypropionic acid derivative compound of formula I 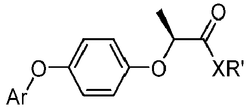 wherein Ar is an aryl group, R' is selected from the group consisting of hydrogen, linear or branched aliphatic, alkoxyalkyl, alicyclic, aralkyl, haloaryl, alkylideneamino-oxyalkyl or heterocyclyl alkyl group, X is selected from NR" or O, and R" is selected from the group consisting of hydrogen, linear or branched aliphatic group, which comprises the reaction in a nonpolar solvent of a phenolpropionic acid derivative compound of formula II 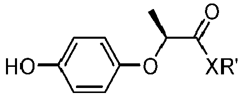 with a haloarene compound of formula Ar-Y when Y is a halogen atom, in the presence of an inorganic or organic base and a catalyst selected from the group of tertiary amines.

This process provides an alternative to the existing processes for the preparation of aryloxyphenoxypropionic acid derivative compounds, and particularly, it provides a solution for the problem of lack of reproducibility in the reaction between haloarene compounds and phenolpropionic acid derivatives in nonpolar solvents in the presence of a catalyst. Surprisingly, we have found that tertiary amine catalysts provide a good yield, excellent optical purity of the R-isomer and reproducibility in the reaction between haloarene compounds and phenolpropionic acid derivatives in nonpolar solvents.

The reaction of phenolpropionic acid derivative compound of formula II with a haloarene compound of formula Ar-Y yields the product aryloxyphenoxypropionic acid derivative compound of formula I and hydrogen halide HY, which is neutralized with the inorganic or organic base to produce a salt as byproduct. In the case of inorganic bases, water is also produced. The so obtained water is removed from the reaction in order to increase the yield of the reaction and to avoid undesired hydrolysis reactions. Water may be removed by azeotropic distillation either under normal pressure or under reduced pressure, or with the aid of an inert gas current.

The process for the preparation of an aryloxyphenoxypropionic acid derivative compound of formula I may include a subsequent step of removal of the salt obtained as byproduct in order to increase the purity of the aryloxyphenoxypropionic acid derivative compound. The salt may be removed by the conventional methods known by the person skilled in the art, including but not limited to filtration, washing the reaction product with water or a mixture of water and a polar solvent in order to dissolve the salt, or by crystallization of the aryloxyphenoxypropionic acid derivative compound in the reaction solvent and subsequent removal of this solvent which contains the salt.

The reaction of the process for the preparation of an aryloxyphenoxypropionic acid derivative compound of formula I may be carried out under an inert atmosphere, wherein the inert gas is nitrogen or argon.

The reaction of the process for the preparation of an aryloxyphenoxypropionic acid derivative compound of formula I may be carried out in a temperature range from 60 to 200 ºC, from 60 to 150 ºC, from 65 to 120ºC, from 70 to 110ºC. In one embodiment, the temperature of the reaction in the process of the invention is the temperature of reflux of the solvent. The reaction of the process for the preparation of an aryloxyphenoxypropionic acid derivative compound of formula I may be carried out in a temperature range from 60 to 200 ºC, from 60 to 150 ºC, from 65 to 120ºC, from 70 to 110ºC, and the reaction may be carried out under an inert atmosphere, wherein the inert gas is nitrogen or argon. The temperature of the reaction in the process of the invention may be the temperature of reflux of the solvent and the reaction may be carried out under an inert atmosphere, wherein the inert gas is nitrogen or argon.

The reaction of the process for the preparation of an aryloxyphenoxypropionic acid derivative compound of formula I may be carried out in a pressure which is normal atmospheric pressure or reduced pressure. In one embodiment, the pressure is reduced pressure in order to facilitate the removal of water produced during the reaction when an inorganic base is used. The reaction of the process for the preparation of an aryloxyphenoxypropionic acid derivative compound of formula I may be carried out in a pressure which is normal atmospheric pressure or reduced pressure, and the temperature of the reaction may be in a range from 60 to 200 ºC, from 60 to 150 ºC, from 65 to 120ºC, from 70 to 110ºC. The reaction of the process for the preparation of an aryloxyphenoxypropionic acid derivative compound of formula I may be carried out in a pressure which is normal atmospheric pressure or reduced pressure, and the temperature of the reaction may be the temperature of reflux of the solvent. The reaction of the process for the preparation of an aryloxyphenoxypropionic acid derivative compound of formula I may be carried out in a pressure which is normal atmospheric pressure or reduced pressure, the temperature of the reaction may be in a range from 60 to 200 ºC, from 60 to 150 ºC, from 65 to 120ºC, from 70 to 110ºC, and the reaction may be carried out under an inert atmosphere, wherein the inert gas is nitrogen or argon. The reaction of the process for the preparation of an aryloxyphenoxypropionic acid derivative compound of formula I may be carried out in a pressure which is normal atmospheric pressure or reduced pressure, the temperature of the reaction may be the temperature of reflux of the solvent, and the reaction may be carried out under an inert atmosphere, wherein the inert gas is nitrogen or argon.

The nonpolar solvent in the reaction of the process for the preparation of an aryloxyphenoxypropionic acid derivative compound of formula I may be a hydrocarbon solvent which optionally contains halogen atoms. The nonpolar solvent in the reaction of the process for the preparation of an aryloxyphenoxypropionic acid derivative compound of formula I may be an aromatic hydrocarbon solvent which optionally contains halogen atoms. The nonpolar solvent in the reaction of the process for the preparation of an aryloxyphenoxypropionic acid derivative compound of formula I may be an aromatic hydrocarbon solvent which optionally contains halogen atoms and selected from the group consisting of toluene, benzene, ethylbenzene, xylene, cumene, cymene, mesitylene, biphenyl, chlorobenzene or bromobenzene.

The reaction of the process for the preparation of an aryloxyphenoxypropionic acid derivative compound of formula I may further comprise an amount of a polar solvent to enhance the solubility of the inorganic or organic base. The amount of the optionally additional polar solvent may range from 0.001% to 10%, from 0.01% to 5%, from 0.1% to 2% by weight of the weight of the nonpolar solvent.

The optionally additional polar solvent in the reaction of the process for the preparation of an aryloxyphenoxypropionic acid derivative compound of formula I may be a polar aprotic solvent. The optionally additional polar solvent in the reaction of the process for the preparation of an aryloxyphenoxypropionic acid derivative compound of formula I may be a polar aprotic solvent selected from the group consisting of ketones, nitriles, alkyl lactams, lactones, sulfoxides, sulfones, nitro compounds, urea derivatives or amides. The optionally additional polar solvent in the reaction of the process for the preparation of an aryloxyphenoxypropionic acid derivative compound of formula I may be a polar aprotic solvent selected from the group consisting of acetone, methyl isobutyl ketone, methyl ethyl ketone, benzonitrile, acetonitrile, N-methyl-2-pyrrolidone, butyrolactone, dimethyl formamide, dimethyl acetamide, dimethyl sulfoxide, sulfolane, nitromethane, nitrobenzene, tetramethyl urea, 1,3-dimethyl-2-imidazolidinone or N-N'-dimethylpropylene urea.

The optionally additional polar solvent in the reaction of the process for the preparation of an aryloxyphenoxypropionic acid derivative compound of formula I may be a polar aprotic solvent, and its amount may range from 0.001% to 10% by weight of the weight of the nonpolar solvent. The optionally additional polar solvent in the reaction of the process for the preparation of an aryloxyphenoxypropionic acid derivative compound of formula I may be a polar aprotic solvent selected from the group consisting of ketones, nitriles, alkyl lactams, lactones, sulfoxides, sulfones, nitro compounds, urea derivatives or amides, and its amount may range from 0.01% to 5% by weight of the weight of the nonpolar solvent. The optionally additional polar solvent in the reaction of the process for the preparation of an aryloxyphenoxypropionic acid derivative compound of formula I may be a polar aprotic solvent selected from the group consisting of acetone, methyl isobutyl ketone, methyl ethyl ketone, benzonitrile, acetonitrile, N-methyl-2-pyrrolidone, butyrolactone, dimethyl formamide, dimethyl acetamide, dimethyl sulfoxide, sulfolane, nitromethane, nitrobenzene, tetramethyl urea, 1,3-dimethyl-2-imidazolidinone or N-N'-dimethylpropylene urea, and its amount may range from 0.1% to 2% by weight of the weight of the nonpolar solvent.

The optionally additional polar solvent in the reaction of the process for the preparation of an aryloxyphenoxypropionic acid derivative compound of formula I may be a polar aprotic solvent, its amount may range from 0.001% to 10% by weight of the weight of the nonpolar solvent, and the nonpolar solvent may be a hydrocarbon solvent which optionally contains halogen atoms. The optionally additional polar solvent in the reaction of the process for the preparation of an aryloxyphenoxypropionic acid derivative compound of formula I may be a polar aprotic solvent selected from the group consisting of ketones, nitriles, alkyl lactams, lactones, sulfoxides, sulfones, nitro compounds, urea derivatives or amides, its amount may range from 0.01% to 5% by weight of the weight of the nonpolar solvent, and the nonpolar solvent may be an aromatic hydrocarbon solvent which optionally contains halogen atoms. The optionally additional polar solvent in the reaction of the process for the preparation of an aryloxyphenoxypropionic acid derivative compound of formula I may be a polar aprotic solvent selected from the group consisting of acetone, methyl isobutyl ketone, methyl ethyl ketone, benzonitrile, acetonitrile, N-methyl-2-pyrrolidone, butyrolactone, dimethyl formamide, dimethyl acetamide, dimethyl sulfoxide, sulfolane, nitromethane, nitrobenzene, tetramethyl urea, 1,3-dimethyl-2-imidazolidinone or N-N'-dimethylpropylene urea, its amount may range from 0.1% to 2% by weight of the weight of the nonpolar solvent, and the nonpolar solvent may be an aromatic hydrocarbon solvent which optionally contains halogen atoms and selected from the group consisting of toluene, benzene, ethylbenzene, xylene, cumene, cymene, mesitylene, biphenyl, chlorobenzene or bromobenzene.

The inorganic or organic base of the process of this invention reacts with the hydrogen halide HY formed together with the aryloxyphenoxypropionic acid derivative compound of formula I, to yield a salt as byproduct. In the case of inorganic bases, water is also produced. The base in the process for the preparation of an aryloxyphenoxypropionic acid derivative compound of formula I may be an inorganic base. The base in the process for the preparation of an aryloxyphenoxypropionic acid derivative compound of formula I may be an inorganic base selected from the group consisting of an alkali metal, alkaline earth metal or ammonium carbonate, bicarbonate, phosphate, hydrogen- or dihydrogenphosphate. The base in the process for the preparation of an aryloxyphenoxypropionic acid derivative compound of formula I may be an inorganic base selected from the group consisting of an alkali metal or alkaline earth metal carbonate or bicarbonate. The base in the process for the preparation of an aryloxyphenoxypropionic acid derivative compound of formula I may be an inorganic base selected from the group consisting of sodium or potassium carbonate or bicarbonate.

The amount of the inorganic or organic base in the process for the preparation of an aryloxyphenoxypropionic acid derivative compound of formula I may range from 1 to 5, from 1 to 3, from 1 to 2 moles by mole of the haloarene compound of formula Ar-Y.

The base in the process for the preparation of an aryloxyphenoxypropionic acid derivative compound of formula I may be an inorganic base, and its amount may range from 1 to 5 moles by mole of the haloarene compound of formula Ar-Y. The base in the process for the preparation of an aryloxyphenoxypropionic acid derivative compound of formula I may be an inorganic base selected from the group consisting of an alkali metal, alkaline earth metal or ammonium carbonate, bicarbonate, phosphate, hydrogen- or dihydrogenphosphate, and its amount may range from 1 to 3 moles by mole of the haloarene compound of formula Ar-Y. The base in the process for the preparation of an aryloxyphenoxypropionic acid derivative compound of formula I may be an inorganic base selected from the group consisting of an alkali metal or alkaline earth metal carbonate or bicarbonate, and its amount may range from 1 to 2 moles by mole of the haloarene compound of formula Ar-Y. The base in the process for the preparation of an aryloxyphenoxypropionic acid derivative compound of formula I may be an inorganic base selected from the group consisting of sodium or potassium carbonate or bicarbonate, and its amount may range from 1 to 2 moles by mole of the haloarene compound of formula Ar-Y.

The base in the process for the preparation of an aryloxyphenoxypropionic acid derivative compound of formula I may be an inorganic or organic base, and the nonpolar solvent in the reaction of the process may be a hydrocarbon solvent which optionally contains halogen atoms. The base in the process for the preparation of an aryloxyphenoxypropionic acid derivative compound of formula I may be an inorganic base, and the nonpolar solvent in the reaction of the process may be a hydrocarbon solvent which optionally contains halogen atoms. The base in the process for the preparation of an aryloxyphenoxypropionic acid derivative compound of formula I may be an inorganic base, and the nonpolar solvent in the reaction may be an aromatic hydrocarbon solvent which optionally contains halogen atoms. The base in the process for the preparation of an aryloxyphenoxypropionic acid derivative compound of formula I may be an inorganic base selected from the group consisting of an alkali metal, alkaline earth metal or ammonium carbonate, bicarbonate, phosphate, hydrogen- or dihydrogenphosphate, and the nonpolar solvent in the reaction of the process may be an aromatic hydrocarbon solvent which optionally contains halogen atoms. The base in the process for the preparation of an aryloxyphenoxypropionic acid derivative compound of formula I may be an inorganic base selected from the group consisting of an alkali metal or alkaline earth metal carbonate or bicarbonate, and the nonpolar solvent in the reaction of the process may be an aromatic hydrocarbon solvent which optionally contains halogen atoms. The base in the process for the preparation of an aryloxyphenoxypropionic acid derivative compound of formula I may be an inorganic base selected from the group consisting of sodium or potassium carbonate or bicarbonate, and the nonpolar solvent in the reaction of the process may be an aromatic hydrocarbon solvent which optionally contains halogen atoms and selected from the group consisting of toluene, benzene, ethylbenzene, xylene, cumene, cymene, mesitylene, biphenyl, chlorobenzene or bromobenzene.

The base in the process for the preparation of an aryloxyphenoxypropionic acid derivative compound of formula I may be an inorganic base, its amount may range from 1 to 5 moles by mole of the haloarene compound of formula Ar-Y, and the nonpolar solvent in the reaction of the process may be a hydrocarbon solvent which optionally contains halogen atoms. The base in the process for the preparation of an aryloxyphenoxypropionic acid derivative compound of formula I may be an inorganic base selected from the group consisting of an alkali metal, alkaline earth metal or ammonium carbonate, bicarbonate, phosphate, hydrogen- or dihydrogenphosphate, its amount may range from 1 to 3 moles by mole of the haloarene compound of formula Ar-Y, and the nonpolar solvent in the reaction of the process may be an aromatic hydrocarbon solvent which optionally contains halogen atoms. The base in the process for the preparation of an aryloxyphenoxypropionic acid derivative compound of formula I may be an inorganic base selected from the group consisting of an alkali metal or alkaline earth metal carbonate or bicarbonate, its amount may range from 1 to 2 moles by mole of the haloarene compound of formula Ar-Y, and the nonpolar solvent in the reaction of the process may be an aromatic hydrocarbon solvent which optionally contains halogen atoms and selected from the group consisting of toluene, benzene, ethylbenzene, xylene, cumene, cymene, mesitylene, biphenyl, chlorobenzene or bromobenzene. The base in the process for the preparation of an aryloxyphenoxypropionic acid derivative compound of formula I may be an inorganic base selected from the group consisting of sodium or potassium carbonate or bicarbonate, its amount may range from 1 to 2 moles by mole of the haloarene compound of formula Ar-Y, and the nonpolar solvent in the reaction of the process may be an aromatic hydrocarbon solvent which optionally contains halogen atoms and selected from the group consisting of toluene, benzene, ethylbenzene, xylene, cumene, cymene, mesitylene, biphenyl, chlorobenzene or bromobenzene.

The base in the process for the preparation of an aryloxyphenoxypropionic acid derivative compound of formula I may be an inorganic base, its amount may range from 1 to 5 moles by mole of the haloarene compound of formula Ar-Y, the nonpolar solvent in the reaction of the process may be a hydrocarbon solvent which optionally contains halogen atoms, and the optionally additional polar solvent in the reaction of the process may be a polar aprotic solvent. The base in the process for the preparation of an aryloxyphenoxypropionic acid derivative compound of formula I may be an inorganic base selected from the group consisting of an alkali metal, alkaline earth metal or ammonium carbonate, bicarbonate, phosphate, hydrogen- or dihydrogenphosphate, its amount may range from 1 to 3 moles by mole of the haloarene compound of formula Ar-Y, the nonpolar solvent in the reaction of the process may be an aromatic hydrocarbon solvent which optionally contains halogen atoms, and the optionally additional polar solvent in the reaction of the process may be a polar aprotic solvent selected from the group consisting of ketones, nitriles, alkyl lactams, lactones, sulfoxides, sulfones, nitro compounds, urea derivatives or amides. The base in the process for the preparation of an aryloxyphenoxypropionic acid derivative compound of formula I may be an inorganic base selected from the group consisting of an alkali metal or alkaline earth metal carbonate or bicarbonate, its amount may range from 1 to 2 moles by mole of the haloarene compound of formula Ar-Y, the nonpolar solvent in the reaction of the process may be an aromatic hydrocarbon solvent which optionally contains halogen atoms and selected from the group consisting of toluene, benzene, ethylbenzene, xylene, cumene, cymene, mesitylene, biphenyl, chlorobenzene or bromobenzene, and the optionally additional polar solvent in the reaction of the process may be a polar aprotic solvent selected from the group consisting of acetone, methyl isobutyl ketone, methyl ethyl ketone, benzonitrile, acetonitrile, N-methyl-2-pyrrolidone, butyrolactone, dimethyl formamide, dimethyl acetamide, dimethyl sulfoxide, sulfolane, nitromethane, nitrobenzene, tetramethyl urea, 1,3-dimethyl-2-imidazolidinone or N-N'-dimethylpropylene urea. The base in the process for the preparation of an aryloxyphenoxypropionic acid derivative compound of formula I may be an inorganic base selected from the group consisting of sodium or potassium carbonate or bicarbonate, its amount may range from 1 to 2 moles by mole of the haloarene compound of formula Ar-Y, the nonpolar solvent in the reaction of the process may be an aromatic hydrocarbon solvent which optionally contains halogen atoms and selected from the group consisting of toluene, benzene, ethylbenzene, xylene, cumene, cymene, mesitylene, biphenyl, chlorobenzene or bromobenzene, and the optionally additional polar solvent in the reaction of the process may be a polar aprotic solvent selected from the group consisting of acetone, methyl isobutyl ketone, methyl ethyl ketone, benzonitrile, acetonitrile, N-methyl-2-pyrrolidone, butyrolactone, dimethyl formamide, dimethyl acetamide, dimethyl sulfoxide, sulfolane, nitromethane, nitrobenzene, tetramethyl urea, 1,3-dimethyl-2-imidazolidinone or N-N'-dimethylpropylene urea.

The base in the process for the preparation of an aryloxyphenoxypropionic acid derivative compound of formula I may be an inorganic base, its amount may range from 1 to 5 moles by mole of the haloarene compound of formula Ar-Y, the nonpolar solvent in the reaction of the process may be a hydrocarbon solvent which optionally contains halogen atoms, the optionally additional polar solvent in the reaction of the process may be a polar aprotic solvent, and its amount may range from 0.001% to 10% by weight of the weight of the nonpolar solvent. The base in the process for the preparation of an aryloxyphenoxypropionic acid derivative compound of formula I may be an inorganic base selected from the group consisting of an alkali metal, alkaline earth metal or ammonium carbonate, bicarbonate, phosphate, hydrogen- or dihydrogenphosphate, its amount may range from 1 to 3 moles by mole of the haloarene compound of formula Ar-Y, the nonpolar solvent in the reaction of the process may be an aromatic hydrocarbon solvent which optionally contains halogen atoms, the optionally additional polar solvent in the reaction of the process may be a polar aprotic solvent selected from the group consisting of ketones, nitriles, alkyl lactams, lactones, sulfoxides, sulfones, nitro compounds, urea derivatives or amides, and its amount may range from 0.01% to 5% by weight of the weight of the nonpolar solvent. The base in the process for the preparation of an aryloxyphenoxypropionic acid derivative compound of formula I may be an inorganic base selected from the group consisting of an alkali metal or alkaline earth metal carbonate or bicarbonate, its amount may range from 1 to 2 moles by mole of the haloarene compound of formula Ar-Y, the nonpolar solvent in the reaction of the process may be an aromatic hydrocarbon solvent which optionally contains halogen atoms and selected from the group consisting of toluene, benzene, ethylbenzene, xylene, cumene, cymene, mesitylene, biphenyl, chlorobenzene or bromobenzene, the optionally additional polar solvent in the reaction of the process may be a polar aprotic solvent selected from the group consisting of acetone, methyl isobutyl ketone, methyl ethyl ketone, benzonitrile, acetonitrile, N-methyl-2-pyrrolidone, butyrolactone, dimethyl formamide, dimethyl acetamide, dimethyl sulfoxide, sulfolane, nitromethane, nitrobenzene, tetramethyl urea, 1,3-dimethyl-2-imidazolidinone or N-N'-dimethylpropylene urea, and its amount may range from 0.1% to 2% by weight of the weight of the nonpolar solvent. The base in the process for the preparation of an aryloxyphenoxypropionic acid derivative compound of formula I may be an inorganic base selected from the group consisting of sodium or potassium carbonate or bicarbonate, its amount may range from 1 to 2 moles by mole of the haloarene compound of formula Ar-Y, the nonpolar solvent in the reaction of the process may be an aromatic hydrocarbon solvent which optionally contains halogen atoms and selected from the group consisting of toluene, benzene, ethylbenzene, xylene, cumene, cymene, mesitylene, biphenyl, chlorobenzene or bromobenzene, the optionally additional polar solvent in the reaction of the process may be a polar aprotic solvent selected from the group consisting of acetone, methyl isobutyl ketone, methyl ethyl ketone, benzonitrile, acetonitrile, N-methyl-2-pyrrolidone, butyrolactone, dimethyl formamide, dimethyl acetamide, dimethyl sulfoxide, sulfolane, nitromethane, nitrobenzene, tetramethyl urea, 1,3-dimethyl-2-imidazolidinone or N-N'-dimethylpropylene urea, and its amount may range from 0.1% to 2% by weight of the weight of the nonpolar solvent.

The catalyst in the reaction of the process for the preparation of an aryloxyphenoxypropionic acid derivative compound of formula I is a tertiary amine. The catalyst in the reaction of the process for the preparation of an aryloxyphenoxypropionic acid derivative compound of formula I may be a bridged bicyclic tertiary amine. The catalyst in the reaction of the process for the preparation of an aryloxyphenoxypropionic acid derivative compound of formula I may be an aliphatic bridged bicyclic tertiary amine. The catalyst in the reaction of the process for the preparation of an aryloxyphenoxypropionic acid derivative compound of formula I may be an aliphatic bridged bicyclic tertiary amine selected from the group consisting of 1,4-diazabicyclo[2.2.2.]octane (i.e. DABCO), quinuclidine or tropane. The catalyst in the reaction of the process for the preparation of an aryloxyphenoxypropionic acid derivative compound of formula I may be DABCO.

The amount of catalyst in the reaction of the process for the preparation of an aryloxyphenoxypropionic acid derivative compound of formula I may range from 0.01% to 50%, from 0.1% to 35%, from 1% to 30% by mole of the moles of the haloarene compound of formula Ar-Y.

The catalyst in the reaction of the process for the preparation of an aryloxyphenoxypropionic acid derivative compound of formula I may be a bridged bicyclic tertiary amine, and its amount may range from 0.01% to 50% by mole of the moles of the haloarene compound of formula Ar-Y. The catalyst in the reaction of the process for the preparation of an aryloxyphenoxypropionic acid derivative compound of formula I may be an aliphatic bridged bicyclic tertiary amine, and its amount may range from 0.1% to 35% by mole of the moles of the haloarene compound of formula Ar-Y. The catalyst in the reaction of the process for the preparation of an aryloxyphenoxypropionic acid derivative compound of formula I may be an aliphatic bridged bicyclic tertiary amine selected from the group consisting of 1,4-diazabicyclo[2.2.2.]octane (i.e. DABCO), quinuclidine or tropane, and its amount may range from 1% to 30% by mole of the moles of the haloarene compound of formula Ar-Y. The catalyst in the reaction of the process for the preparation of an aryloxyphenoxypropionic acid derivative compound of formula I may be DABCO, and its amount may range from 1% to 30% by mole of the moles of the haloarene compound of formula Ar-Y.

The catalyst in the reaction of the process for the preparation of an aryloxyphenoxypropionic acid derivative compound of formula I is a tertiary amine, and the base in the process may be an inorganic base. The catalyst in the reaction of the process for the preparation of an aryloxyphenoxypropionic acid derivative compound of formula I may be a bridged bicyclic tertiary amine, and the base in the process may be an inorganic base selected from the group consisting of an alkali metal, alkaline earth metal or ammonium carbonate, bicarbonate, phosphate, hydrogen- or dihydrogenphosphate. The catalyst in the reaction of the process for the preparation of an aryloxyphenoxypropionic acid derivative compound of formula I may be an aliphatic bridged bicyclic tertiary amine, and the base in the process may be an inorganic base selected from the group consisting of an alkali metal or alkaline earth metal carbonate or bicarbonate. The catalyst in the reaction of the process for the preparation of an aryloxyphenoxypropionic acid derivative compound of formula I may be an aliphatic bridged bicyclic tertiary amine selected from the group consisting of 1,4-diazabicyclo[2.2.2.]octane (i.e. DABCO), quinuclidine or tropane, and the base in the process may be an inorganic base selected from the group consisting of sodium or potassium carbonate or bicarbonate. The catalyst in the reaction of the process for the preparation of an aryloxyphenoxypropionic acid derivative compound of formula I may be DABCO and the base in the process may be an inorganic base selected from the group consisting of sodium or potassium carbonate.

The catalyst in the reaction of the process for the preparation of an aryloxyphenoxypropionic acid derivative compound of formula I is a tertiary amine, its amount may range from 0.01% to 50% by mole of the moles of the haloarene compound of formula Ar-Y, the base in the process may be an inorganic base, and its amount may range from 1 to 5 moles by mole of the haloarene compound of formula Ar-Y. The catalyst in the reaction of the process for the preparation of an aryloxyphenoxypropionic acid derivative compound of formula I may be a bridged bicyclic tertiary amine, its amount may range from 0.01% to 50% by mole of the moles of the haloarene compound of formula Ar-Y, the base in the process may be an inorganic base selected from the group consisting of an alkali metal, alkaline earth metal or ammonium carbonate, bicarbonate, phosphate, hydrogen- or dihydrogenphosphate, and its amount may range from 1 to 5 moles by mole of the haloarene compound of formula Ar-Y. The catalyst in the reaction of the process for the preparation of an aryloxyphenoxypropionic acid derivative compound of formula I may be an aliphatic bridged bicyclic tertiary amine, its amount may range from 0.1% to 35% by mole of the moles of the haloarene compound of formula Ar-Y, the base in the process may be an inorganic base selected from the group consisting of an alkali metal or alkaline earth metal carbonate or bicarbonate, and its amount may range from 1 to 3 moles by mole of the haloarene compound of formula Ar-Y. The catalyst in the reaction of the process for the preparation of an aryloxyphenoxypropionic acid derivative compound of formula I may be an aliphatic bridged bicyclic tertiary amine selected from the group consisting of 1,4-diazabicyclo[2.2.2.]octane (i.e. DABCO), quinuclidine or tropane, its amount may range from 1% to 30% by mole of the moles of the haloarene compound of formula Ar-Y, the base in the process may be an inorganic base selected from the group consisting of sodium or potassium carbonate or bicarbonate, and its amount may range from 1 to 2 moles by mole of the haloarene compound of formula Ar-Y. The catalyst in the reaction of the process for the preparation of an aryloxyphenoxypropionic acid derivative compound of formula I may be DABCO, its amount may range from 1% to 30% by mole of the moles of the haloarene compound of formula Ar-Y, the base in the process may be an inorganic base selected from the group consisting of sodium or potassium carbonate, and its amount may range from 1 to 2 moles by mole of the haloarene compound of formula Ar-Y.

The catalyst in the reaction of the process for the preparation of an aryloxyphenoxypropionic acid derivative compound of formula I is a tertiary amine, the base in the process may be an inorganic base, and the nonpolar solvent in the reaction of the process may be a hydrocarbon solvent which optionally contains halogen atoms. The catalyst in the reaction of the process for the preparation of an aryloxyphenoxypropionic acid derivative compound of formula I may be a bridged bicyclic tertiary amine, the base in the process may be an inorganic base selected from the group consisting of an alkali metal, alkaline earth metal or ammonium carbonate, bicarbonate, phosphate, hydrogen- or dihydrogenphosphate, and the nonpolar solvent in the reaction of the process may be a hydrocarbon solvent which optionally contains halogen atoms. The catalyst in the reaction of the process for the preparation of an aryloxyphenoxypropionic acid derivative compound of formula I may be an aliphatic bridged bicyclic tertiary amine, the base in the process may be an inorganic base selected from the group consisting of an alkali metal or alkaline earth metal carbonate or bicarbonate, and the nonpolar solvent in the reaction of the process may be an aromatic hydrocarbon solvent which optionally contains halogen atoms. The catalyst in the reaction of the process for the preparation of an aryloxyphenoxypropionic acid derivative compound of formula I may be an aliphatic bridged bicyclic tertiary amine selected from the group consisting of 1,4-diazabicyclo[2.2.2.]octane (i.e. DABCO), quinuclidine or tropane, the base in the process may be an inorganic base selected from the group consisting of sodium or potassium carbonate or bicarbonate, and the nonpolar solvent in the reaction of the process may be an aromatic hydrocarbon solvent which optionally contains halogen atoms. The catalyst in the reaction of the process for the preparation of an aryloxyphenoxypropionic acid derivative compound of formula I may be DABCO, the base in the process may be an inorganic base selected from the group consisting of sodium or potassium carbonate, and the nonpolar solvent in the reaction of the process may be an aromatic hydrocarbon solvent which optionally contains halogen atoms and selected from the group consisting of toluene, benzene, ethylbenzene, xylene, cumene, cymene, mesitylene, biphenyl, chlorobenzene or bromobenzene.

The catalyst in the reaction of the process for the preparation of an aryloxyphenoxypropionic acid derivative compound of formula I is a tertiary amine, its amount may range from 0.01% to 50% by mole of the moles of the haloarene compound of formula Ar-Y, the base in the process may be an inorganic base, its amount may range from 1 to 5 moles by mole of the haloarene compound of formula Ar-Y, and the nonpolar solvent in the reaction of the process may be a hydrocarbon solvent which optionally contains halogen atoms. The catalyst in the reaction of the process for the preparation of an aryloxyphenoxypropionic acid derivative compound of formula I may be a bridged bicyclic tertiary amine, its amount may range from 0.01% to 50% by mole of the moles of the haloarene compound of formula Ar-Y, the base in the process may be an inorganic base selected from the group consisting of an alkali metal, alkaline earth metal or ammonium carbonate, bicarbonate, phosphate, hydrogen- or dihydrogenphosphate, its amount may range from 1 to 5 moles by mole of the haloarene compound of formula Ar-Y, and the nonpolar solvent in the reaction of the process may be a hydrocarbon solvent which optionally contains halogen atoms. The catalyst in the reaction of the process for the preparation of an aryloxyphenoxypropionic acid derivative compound of formula I may be an aliphatic bridged bicyclic tertiary amine, its amount may range from 0.1% to 35% by mole of the moles of the haloarene compound of formula Ar-Y, the base in the process may be an inorganic base selected from the group consisting of an alkali metal or alkaline earth metal carbonate or bicarbonate, its amount may range from 1 to 3 moles by mole of the haloarene compound of formula Ar-Y, and the nonpolar solvent in the reaction of the process may be an aromatic hydrocarbon solvent which optionally contains halogen atoms. The catalyst in the reaction of the process for the preparation of an aryloxyphenoxypropionic acid derivative compound of formula I may be an aliphatic bridged bicyclic tertiary amine selected from the group consisting of 1,4-diazabicyclo[2.2.2.]octane (i.e. DABCO), quinuclidine or tropane, its amount may range from 1% to 30% by mole of the moles of the haloarene compound of formula Ar-Y, the base in the process may be an inorganic base selected from the group consisting of sodium or potassium carbonate or bicarbonate, its amount may range from 1 to 2 moles by mole of the haloarene compound of formula Ar-Y, and the nonpolar solvent in the reaction of the process may be an aromatic hydrocarbon solvent which optionally contains halogen atoms. The catalyst in the reaction of the process for the preparation of an aryloxyphenoxypropionic acid derivative compound of formula I may be DABCO, its amount may range from 1% to 30% by mole of the moles of the haloarene compound of formula Ar-Y, the base in the process may be an inorganic base selected from the group consisting of sodium or potassium carbonate, and its amount may range from 1 to 2 moles by mole of the haloarene compound of formula Ar-Y, and the nonpolar solvent in the reaction of the process may be an aromatic hydrocarbon solvent which optionally contains halogen atoms and selected from the group consisting of toluene, benzene, ethylbenzene, xylene, cumene, cymene, mesitylene, biphenyl, chlorobenzene or bromobenzene.

The catalyst in the reaction of the process for the preparation of an aryloxyphenoxypropionic acid derivative compound of formula I is a tertiary amine, its amount may range from 0.01% to 50% by mole of the moles of the haloarene compound of formula Ar-Y, the base in the process may be an inorganic base, its amount may range from 1 to 5 moles by mole of the haloarene compound of formula Ar-Y, the nonpolar solvent in the reaction of the process may be a hydrocarbon solvent which optionally contains halogen atoms, and the optionally additional polar solvent in the reaction of the process may be a polar aprotic solvent. The catalyst in the reaction of the process for the preparation of an aryloxyphenoxypropionic acid derivative compound of formula I may be a bridged bicyclic tertiary amine, its amount may range from 0.01% to 50% by mole of the moles of the haloarene compound of formula Ar-Y, the base in the process may be an inorganic base selected from the group consisting of an alkali metal, alkaline earth metal or ammonium carbonate, bicarbonate, phosphate, hydrogen- or dihydrogenphosphate, its amount may range from 1 to 5 moles by mole of the haloarene compound of formula Ar-Y, the nonpolar solvent in the reaction of the process may be a hydrocarbon solvent which optionally contains halogen atoms, and the optionally additional polar solvent in the reaction of the process may be a polar aprotic solvent. The catalyst in the reaction of the process for the preparation of an aryloxyphenoxypropionic acid derivative compound of formula I may be an aliphatic bridged bicyclic tertiary amine, its amount may range from 0.1% to 35% by mole of the moles of the haloarene compound of formula Ar-Y, the base in the process may be an inorganic base selected from the group consisting of an alkali metal or alkaline earth metal carbonate or bicarbonate, its amount may range from 1 to 3 moles by mole of the haloarene compound of formula Ar-Y, the nonpolar solvent in the reaction of the process may be an aromatic hydrocarbon solvent which optionally contains halogen atoms, and the optionally additional polar solvent in the reaction of the process may be a polar aprotic solvent selected from the group consisting of ketones, nitriles, alkyl lactams, lactones, sulfoxides, sulfones, nitro compounds, urea derivatives or amides. The catalyst in the reaction of the process for the preparation of an aryloxyphenoxypropionic acid derivative compound of formula I may be an aliphatic bridged bicyclic tertiary amine selected from the group consisting of 1,4-diazabicyclo[2.2.2.]octane (i.e. DABCO), quinuclidine or tropane, its amount may range from 1% to 30% by mole of the moles of the haloarene compound of formula Ar-Y, the base in the process may be an inorganic base selected from the group consisting of sodium or potassium carbonate or bicarbonate, its amount may range from 1 to 2 moles by mole of the haloarene compound of formula Ar-Y, and the nonpolar solvent in the reaction of the process may be an aromatic hydrocarbon solvent which optionally contains halogen atoms, and the optionally additional polar solvent in the reaction of the process may be a polar aprotic solvent selected from the group consisting of acetone, methyl isobutyl ketone, methyl ethyl ketone, benzonitrile, acetonitrile, N-methyl-2-pyrrolidone, butyrolactone, dimethyl formamide, dimethyl acetamide, dimethyl sulfoxide, sulfolane, nitromethane, nitrobenzene, tetramethyl urea, 1,3-dimethyl-2-imidazolidinone or N-N'-dimethylpropylene urea. The catalyst in the reaction of the process for the preparation of an aryloxyphenoxypropionic acid derivative compound of formula I may be DABCO, its amount may range from 1% to 30% by mole of the moles of the haloarene compound of formula Ar-Y, the base in the process may be an inorganic base selected from the group consisting of sodium or potassium carbonate, its amount may range from 1 to 2 moles by mole of the haloarene compound of formula Ar-Y, the nonpolar solvent in the reaction of the process may be an aromatic hydrocarbon solvent which optionally contains halogen atoms and selected from the group consisting of toluene, benzene, ethylbenzene, xylene, cumene, cymene, mesitylene, biphenyl, chlorobenzene or bromobenzene, and the optionally additional polar solvent in the reaction of the process may be a polar aprotic solvent selected from the group consisting of acetone, methyl isobutyl ketone, methyl ethyl ketone, benzonitrile, acetonitrile, N-methyl-2-pyrrolidone, butyrolactone, dimethyl formamide, dimethyl acetamide, dimethyl sulfoxide, sulfolane, nitromethane, nitrobenzene, tetramethyl urea, 1,3-dimethyl-2-imidazolidinone or N-N'-dimethylpropylene urea.

The catalyst in the reaction of the process for the preparation of an aryloxyphenoxypropionic acid derivative compound of formula I is a tertiary amine, its amount may range from 0.01% to 50% by mole of the moles of the haloarene compound of formula Ar-Y, the base in the process may be an inorganic base, its amount may range from 1 to 5 moles by mole of the haloarene compound of formula Ar-Y, the nonpolar solvent in the reaction of the process may be a hydrocarbon solvent which optionally contains halogen atoms, the optionally additional polar solvent in the reaction of the process may be a polar aprotic solvent, and its amount may range from 0.001% to 10% by weight of the weight of the nonpolar solvent. The catalyst in the reaction of the process for the preparation of an aryloxyphenoxypropionic acid derivative compound of formula I may be a bridged bicyclic tertiary amine, its amount may range from 0.01% to 50% by mole of the moles of the haloarene compound of formula Ar-Y, the base in the process may be an inorganic base selected from the group consisting of an alkali metal, alkaline earth metal or ammonium carbonate, bicarbonate, phosphate, hydrogen- or dihydrogenphosphate, its amount may range from 1 to 5 moles by mole of the haloarene compound of formula Ar-Y, the nonpolar solvent in the reaction of the process may be a hydrocarbon solvent which optionally contains halogen atoms, the optionally additional polar solvent in the reaction of the process may be a polar aprotic solvent, and its amount may range from 0.001% to 10% by weight of the weight of the nonpolar solvent. The catalyst in the reaction of the process for the preparation of an aryloxyphenoxypropionic acid derivative compound of formula I may be an aliphatic bridged bicyclic tertiary amine, its amount may range from 0.1% to 35% by mole of the moles of the haloarene compound of formula Ar-Y, the base in the process may be an inorganic base selected from the group consisting of an alkali metal or alkaline earth metal carbonate or bicarbonate, its amount may range from 1 to 3 moles by mole of the haloarene compound of formula Ar-Y, the nonpolar solvent in the reaction of the process may be an aromatic hydrocarbon solvent which optionally contains halogen atoms, the optionally additional polar solvent in the reaction of the process may be a polar aprotic solvent selected from the group consisting of ketones, nitriles, alkyl lactams, lactones, sulfoxides, sulfones, nitro compounds, urea derivatives or amides, and its amount may range from 0.01% to 5% by weight of the weight of the nonpolar solvent. The catalyst in the reaction of the process for the preparation of an aryloxyphenoxypropionic acid derivative compound of formula I may be an aliphatic bridged bicyclic tertiary amine selected from the group consisting of 1,4-diazabicyclo[2.2.2.]octane (i.e. DABCO), quinuclidine or tropane, its amount may range from 1% to 30% by mole of the moles of the haloarene compound of formula Ar-Y, the base in the process may be an inorganic base selected from the group consisting of sodium or potassium carbonate or bicarbonate, its amount may range from 1 to 2 moles by mole of the haloarene compound of formula Ar-Y, the nonpolar solvent in the reaction of the process may be an aromatic hydrocarbon solvent which optionally contains halogen atoms, the optionally additional polar solvent in the reaction of the process may be a polar aprotic solvent selected from the group consisting of ketones, nitriles, alkyl lactams, lactones, sulfoxides, sulfones, nitro compounds, urea derivatives or amides, and its amount may range from 0.01% to 5% by weight of the weight of the nonpolar solvent. The catalyst in the reaction of the process for the preparation of an aryloxyphenoxypropionic acid derivative compound of formula I may be DABCO, its amount may range from 1% to 30% by mole of the moles of the haloarene compound of formula Ar-Y, the base in the process may be an inorganic base selected from the group consisting of sodium or potassium carbonate, its amount may range from 1 to 2 moles by mole of the haloarene compound of formula Ar-Y, the nonpolar solvent in the reaction of the process may be an aromatic hydrocarbon solvent which optionally contains halogen atoms and selected from the group consisting of toluene, benzene, ethylbenzene, xylene, cumene, cymene, mesitylene, biphenyl, chlorobenzene or bromobenzene, the optionally additional polar solvent in the reaction of the process may be a polar aprotic solvent selected from the group consisting of acetone, methyl isobutyl ketone, methyl ethyl ketone, benzonitrile, acetonitrile, N-methyl-2-pyrrolidone, butyrolactone, dimethyl formamide, dimethyl acetamide, dimethyl sulfoxide, sulfolane, nitromethane, nitrobenzene, tetramethyl urea, 1,3-dimethyl-2-imidazolidinone or N-N'-dimethylpropylene urea, and its amount may range from 0.1% to 2% by weight of the weight of the nonpolar solvent.

The aryl group of the aryloxyphenoxypropionic acid derivative compound of formula I in its process of preparation may be selected from the group consisting of substituted phenyl, substituted 2-pyridinyl, substituted 2-benzoxazolyl, substituted 2-quinoxalinyl, substituted 2-benzothiazolyl, substituted naphthyl or substituted 2-benzimidazole, wherein the substituents are selected from the group consisting of halogen, aliphatic, halo aliphatic, alkoxy, thioalkyl, cyano or nitro groups. The aryl group of the aryloxyphenoxypropionic acid derivative compound of formula I in its process of preparation may be selected from the group consisting of substituted phenyl, substituted 2-pyridinyl, substituted 2-benzoxazolyl or substituted 2-quinoxalinyl, wherein the substituents are selected from the group consisting of halogen, aliphatic, halo aliphatic, alkoxy, thioalkyl, cyano or nitro groups. The aryl group of the aryloxyphenoxypropionic acid derivative compound of formula I in its process of preparation may be selected from the group consisting of halogen substituted 2-quinoxalinyl, halogen substituted 2-benzoxazolyl, halogen and/or haloalkyl substituted 2-pyridinyl, or halogen and/or cyano substituted phenyl. The aryl group of the aryloxyphenoxypropionic acid derivative compound of formula I in its process of preparation may be selected from the group consisting of 6-chloro-2-quinoxalinyl, 6-chloro-2-benzoxazolyl, 3-chloro-5-(trifluoromethyl)-2-pyridinyl, 5-(trifluoromethyl)-2-pyridinyl, 4-cyano-2-fluorophenyl, 5-chloro-3-fluoro-2-pyridinyl or 2,4-dichlorophenyl. The aryl group of the aryloxyphenoxypropionic acid derivative compound of formula I in its process of preparation may be 6-chloro-2-quinoxalinyl.

The aryl group of the haloarene compound of formula Ar-Y may be selected from the group consisting of substituted phenyl, substituted 2-pyridinyl, substituted 2-benzoxazolyl, substituted 2-quinoxalinyl, substituted 2-benzothiazolyl, substituted naphthyl or substituted 2-benzimidazole, wherein the substituents are selected from the group consisting of halogen, aliphatic, halo aliphatic, alkoxy, thioalkyl, cyano or nitro groups, and Y is a halogen atom. The aryl group of the haloarene compound of formula Ar-Y may be selected from the group consisting of substituted phenyl, substituted 2-pyridinyl, substituted 2-benzoxazolyl or substituted 2-quinoxalinyl, wherein the substituents are selected from the group consisting of halogen, aliphatic, halo aliphatic, alkoxy, thioalkyl, cyano or nitro groups, and Y is a halogen atom. The aryl group of the haloarene compound of formula Ar-Y may be selected from the group consisting of halogen substituted 2-quinoxalinyl, halogen substituted 2-benzoxazolyl, halogen and/or haloalkyl substituted 2-pyridinyl, or halogen and/or cyano substituted phenyl, and Y is a halogen atom. The aryl group of the haloarene compound of formula Ar-Y may be selected from the group consisting of 6-chloro-2-quinoxalinyl, 6-chloro-2-benzoxazolyl, 3-chloro-5-(trifluoromethyl)-2-pyridinyl, 5-(trifluoromethyl)-2-pyridinyl, 4-cyano-2-fluorophenyl, 5-chloro-3-fluoro-2-pyridinyl or 2,4-dichlorophenyl, and Y may be selected from chlorine, bromine or iodine. The aryl group of the haloarene compound of formula Ar-Y may be 6-chloro-2-quinoxalinyl, and Y may be selected from chlorine or bromine.

The R' group of the phenolpropionic acid derivative compound of formula II or the aryloxyphenoxypropionic acid derivative compound of formula I may be selected from the group consisting of hydrogen, linear or branched alkyl, alkynyl, alkoxyalkyl, aralkyl, haloaryl, alkylideneamino-oxyalkyl or heterocyclyl alkyl group. The R' group of the phenolpropionic acid derivative compound of formula II or the aryloxyphenoxypropionic acid derivative compound of formula I may be selected from the group consisting of hydrogen, linear or branched alkyl, alkynyl, alkoxyalkyl, benzyl, halophenyl, alkylideneamino-oxyalkyl or heterocyclic-ether alkyl group. The R' group of the phenolpropionic acid derivative compound of formula II or the aryloxyphenoxypropionic acid derivative compound of formula I may be selected from the group consisting of hydrogen, linear or branched alkyl, alkynyl, alkoxyalkyl, 2-halophenyl, alkylideneamino-oxyalkyl or 2-heterocyclic-ether alkyl. The R' group of the phenolpropionic acid derivative compound of formula II or the aryloxyphenoxypropionic acid derivative compound of formula I may be selected from the group consisting of hydrogen, butyl, methyl, ethyl, 2-propynyl, ethoxyethyl, methoxyethyl, 2-fluorophenyl, 2-isopropylideneamino-oxyethyl or (tetrahydro-2-furanyl)methyl. The R' group of the phenolpropionic acid derivative compound of formula II or the aryloxyphenoxypropionic acid derivative compound of formula I may be selected from the group consisting of hydrogen, methyl, ethyl, 2-isopropylideneamino-oxyethyl or (tetrahydro-2-furanyl)methyl.

The X is selected from NR" or O, and R" is selected from the group consisting of hydrogen, linear or branched aliphatic group. The X is selected from NR" or O, and R" may be selected from the group consisting of hydrogen, linear or branched alkyl group. The X is selected from NR" or O, and R" may be linear or branched alkyl group. The X is selected from NR" or O, and R" may be selected from the group consisting of methyl, ethyl, isopropyl or propyl. The X may be O.

The R' group of the phenolpropionic acid derivative compound of formula II or the aryloxyphenoxypropionic acid derivative compound of formula I may be selected from the group consisting of hydrogen, linear or branched alkyl, alkynyl, alkoxyalkyl, aralkyl, haloaryl, alkylideneamino-oxyalkyl or heterocyclyl alkyl group, X is selected from NR" or O, and R" is selected from the group consisting of hydrogen, linear or branched aliphatic group. The R' group of the phenolpropionic acid derivative compound of formula II or the aryloxyphenoxypropionic acid derivative compound of formula I may be selected from the group consisting of hydrogen, linear or branched alkyl, alkynyl, alkoxyalkyl, benzyl, halophenyl, alkylideneamino-oxyalkyl or heterocyclic-ether alkyl group, X is selected from NR" or O, and R" may be selected from the group consisting of hydrogen, linear or branched alkyl group. The R' group of the phenolpropionic acid derivative compound of formula II or the aryloxyphenoxypropionic acid derivative compound of formula I may be selected from the group consisting of hydrogen, linear or branched alkyl, alkynyl, alkoxyalkyl, 2-halophenyl, alkylideneamino-oxyalkyl or 2-heterocyclic-ether alkyl, X is selected from NR" or O, and R" may be linear or branched alkyl group. The R' group of the phenolpropionic acid derivative compound of formula II or the aryloxyphenoxypropionic acid derivative compound of formula I may be selected from the group consisting of hydrogen, butyl, methyl, ethyl, 2-propynyl, ethoxyethyl, methoxyethyl, 2-fluorophenyl, 2-isopropylideneamino-oxyethyl or (tetrahydro-2-furanyl)methyl, X is selected from NR" or O, and R" may be selected from the group consisting of methyl, ethyl, isopropyl or propyl. The R' group of the phenolpropionic acid derivative compound of formula II or the aryloxyphenoxypropionic acid derivative compound of formula I may be selected from the group consisting of hydrogen, methyl, ethyl, 2-isopropylideneamino-oxyethyl or (tetrahydro-2-furanyl)methyl, and X may be O.

The aryl group of the haloarene compound of formula Ar-Y may be selected from the group consisting of substituted phenyl, substituted 2-pyridinyl, substituted 2-benzoxazolyl, substituted 2-quinoxalinyl, substituted 2-benzothiazolyl, substituted naphthyl or substituted 2-benzimidazole, wherein the substituents are selected from the group consisting of halogen, aliphatic, halo aliphatic, alkoxy, thioalkyl, cyano or nitro groups, the R' group of the phenolpropionic acid derivative compound of formula II may be selected from the group consisting of hydrogen, linear or branched alkyl, alkynyl, alkoxyalkyl, aralkyl, haloaryl, alkylideneamino-oxyalkyl or heterocyclyl alkyl group, X is selected from NR" or O, and R" is selected from the group consisting of hydrogen, linear or branched aliphatic group. The aryl group of the haloarene compound of formula Ar-Y may be selected from the group consisting of substituted phenyl, substituted 2-pyridinyl, substituted 2-benzoxazolyl or substituted 2-quinoxalinyl, wherein the substituents are selected from the group consisting of halogen, aliphatic, halo aliphatic, alkoxy, thioalkyl, cyano or nitro groups, the R' group of the phenolpropionic acid derivative compound of formula II may be selected from the group consisting of hydrogen, linear or branched alkyl, alkynyl, alkoxyalkyl, benzyl, halophenyl, alkylideneamino-oxyalkyl or heterocyclic-ether alkyl group, X is selected from NR" or O, and R" may be selected from the group consisting of hydrogen, linear or branched alkyl group. The aryl group of the haloarene compound of formula Ar-Y may be selected from the group consisting of halogen substituted 2-quinoxalinyl, halogen substituted 2-benzoxazolyl, halogen and/or haloalkyl substituted 2-pyridinyl, or halogen and/or cyano substituted phenyl, the R' group of the phenolpropionic acid derivative compound of formula II may be selected from the group consisting of hydrogen, linear or branched alkyl, alkynyl, alkoxyalkyl, 2-halophenyl, alkylideneamino-oxyalkyl or 2-heterocyclic-ether alkyl, X is selected from NR" or O, and R" may be linear or branched alkyl group. The aryl group of the haloarene compound of formula Ar-Y may be selected from the group consisting of 6-chloro-2-quinoxalinyl, 6-chloro-2-benzoxazolyl, 3-chloro-5-(trifluoromethyl)-2-pyridinyl, 5-(trifluoromethyl)-2-pyridinyl, 4-cyano-2-fluorophenyl, 5-chloro-3-fluoro-2-pyridinyl or 2,4-dichlorophenyl, the R' group of the phenolpropionic acid derivative compound of formula II may be selected from the group consisting of hydrogen, butyl, methyl, ethyl, 2-propynyl, ethoxyethyl, methoxyethyl, 2-fluorophenyl, 2-isopropylideneamino-oxyethyl or (tetrahydro-2-furanyl)methyl, X is selected from NR" or O, and R" may be selected from the group consisting of methyl, ethyl, isopropyl or propyl. The aryl group of the haloarene compound of formula Ar-Y may be 6-chloro-2-quinoxalinyl, the R' group of the phenolpropionic acid derivative compound of formula II may be selected from the group consisting of hydrogen, methyl, ethyl, 2-isopropylideneamino-oxyethyl or (tetrahydro-2-furanyl)methyl, and X may be O.

The molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 2.5:1, from 0.9:1 to 2:1, from 0.9:1 to 1.5:1, from 0.9:1 to 1.2:1.

The aryl group of the haloarene compound of formula Ar-Y may be selected from the group consisting of substituted phenyl, substituted 2-pyridinyl, substituted 2-benzoxazolyl, substituted 2-quinoxalinyl, substituted 2-benzothiazolyl, substituted naphthyl or substituted 2-benzimidazole, wherein the substituents are selected from the group consisting of halogen, aliphatic, halo aliphatic, alkoxy, thioalkyl, cyano or nitro groups, the R' group of the phenolpropionic acid derivative compound of formula II may be selected from the group consisting of hydrogen, linear or branched alkyl, alkynyl, alkoxyalkyl, aralkyl, haloaryl, alkylideneamino-oxyalkyl or heterocyclyl alkyl group, X is selected from NR" or O, R" is selected from the group consisting of hydrogen, linear or branched aliphatic group, and the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 2.5:1. The aryl group of the haloarene compound of formula Ar-Y may be selected from the group consisting of substituted phenyl, substituted 2-pyridinyl, substituted 2-benzoxazolyl or substituted 2-quinoxalinyl, wherein the substituents are selected from the group consisting of halogen, aliphatic, halo aliphatic, alkoxy, thioalkyl, cyano or nitro groups, and the R' group of the phenolpropionic acid derivative compound of formula II may be selected from the group consisting of hydrogen, linear or branched alkyl, alkynyl, alkoxyalkyl, benzyl, halophenyl, alkylideneamino-oxyalkyl or heterocyclic-ether alkyl group, X is selected from NR" or O, R" may be selected from the group consisting of hydrogen, linear or branched alkyl group, and the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 2.5:1. The aryl group of the haloarene compound of formula Ar-Y may be selected from the group consisting of halogen substituted 2-quinoxalinyl, halogen substituted 2-benzoxazolyl, halogen and/or haloalkyl substituted 2-pyridinyl, or halogen and/or cyano substituted phenyl, and the R' group of the phenolpropionic acid derivative compound of formula II may be selected from the group consisting of hydrogen, linear or branched alkyl, alkynyl, alkoxyalkyl, 2-halophenyl, alkylideneamino-oxyalkyl or 2-heterocyclic-ether alkyl, X is selected from NR" or O, R" may be linear or branched alkyl group, and the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 2:1. The aryl group of the haloarene compound of formula Ar-Y may be selected from the group consisting of 6-chloro-2-quinoxalinyl, 6-chloro-2-benzoxazolyl, 3-chloro-5-(trifluoromethyl)-2-pyridinyl, 5-(trifluoromethyl)-2-pyridinyl, 4-cyano-2-fluorophenyl, 5-chloro-3-fluoro-2-pyridinyl or 2,4-dichlorophenyl, and the R' group of the phenolpropionic acid derivative compound of formula II may be selected from the group consisting of hydrogen, butyl, methyl, ethyl, 2-propynyl, ethoxyethyl, methoxyethyl, 2-fluorophenyl, 2-isopropylideneamino-oxyethyl or (tetrahydro-2-furanyl)methyl, X is selected from NR" or O, R" may be selected from the group consisting of methyl, ethyl, isopropyl or propyl, and the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 1.5:1. The aryl group of the haloarene compound of formula Ar-Y may be 6-chloro-2-quinoxalinyl, and the R' group of the phenolpropionic acid derivative compound of formula II may be selected from the group consisting of hydrogen, methyl, ethyl, 2-isopropylideneamino-oxyethyl or (tetrahydro-2-furanyl)methyl), X may be O, and the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 1.2:1.

The aryl group of the haloarene compound of formula Ar-Y may be selected from the group consisting of substituted phenyl, substituted 2-pyridinyl, substituted 2-benzoxazolyl, substituted 2-quinoxalinyl, substituted 2-benzothiazolyl, substituted naphthyl or substituted 2-benzimidazole, wherein the substituents are selected from the group consisting of halogen, aliphatic, halo aliphatic, alkoxy, thioalkyl, cyano or nitro groups, the R' group of the phenolpropionic acid derivative compound of formula II may be selected from the group consisting of hydrogen, linear or branched alkyl, alkynyl, alkoxyalkyl, aralkyl, haloaryl, alkylideneamino-oxyalkyl or heterocyclyl alkyl group, X is selected from NR" or O, R" is selected from the group consisting of hydrogen, linear or branched aliphatic group, and the catalyst in the reaction of the process for the preparation of an aryloxyphenoxypropionic acid derivative compound of formula I is a tertiary amine. The aryl group of the haloarene compound of formula Ar-Y may be selected from the group consisting of substituted phenyl, substituted 2-pyridinyl, substituted 2-benzoxazolyl or substituted 2-quinoxalinyl, wherein the substituents are selected from the group consisting of halogen, aliphatic, halo aliphatic, alkoxy, thioalkyl, cyano or nitro groups, the R' group of the phenolpropionic acid derivative compound of formula II may be selected from the group consisting of hydrogen, linear or branched alkyl, alkynyl, alkoxyalkyl, benzyl, halophenyl, alkylideneamino-oxyalkyl or heterocyclic-ether alkyl group, X is selected from NR" or O, R" may be selected from the group consisting of hydrogen, linear or branched alkyl group, and the catalyst in the reaction of the process for the preparation of an aryloxyphenoxypropionic acid derivative compound of formula I may be a bridged bicyclic tertiary amine. The aryl group of the haloarene compound of formula Ar-Y may be selected from the group consisting of halogen substituted 2-quinoxalinyl, halogen substituted 2-benzoxazolyl, halogen and/or haloalkyl substituted 2-pyridinyl, or halogen and/or cyano substituted phenyl, the R' group of the phenolpropionic acid derivative compound of formula II may be selected from the group consisting of hydrogen, linear or branched alkyl, alkynyl, alkoxyalkyl, 2-halophenyl, alkylideneamino-oxyalkyl or 2-heterocyclic-ether alkyl, X is selected from NR" or O, R" may be linear or branched alkyl group, and the catalyst in the reaction of the process for the preparation of an aryloxyphenoxypropionic acid derivative compound of formula I may be an aliphatic bridged bicyclic tertiary amine. The aryl group of the haloarene compound of formula Ar-Y may be selected from the group consisting of 6-chloro-2-quinoxalinyl, 6-chloro-2-benzoxazolyl, 3-chloro-5-(trifluoromethyl)-2-pyridinyl, 5-(trifluoromethyl)-2-pyridinyl, 4-cyano-2-fluorophenyl, 5-chloro-3-fluoro-2-pyridinyl or 2,4-dichlorophenyl, the R' group of the phenolpropionic acid derivative compound of formula II may be selected from the group consisting of hydrogen, butyl, methyl, ethyl, 2-propynyl, ethoxyethyl, methoxyethyl, 2-fluorophenyl, 2-isopropylideneamino-oxyethyl or (tetrahydro-2-furanyl)methyl, X is selected from NR" or O, R" may be selected from the group consisting of methyl, ethyl, isopropyl or propyl, and the catalyst in the reaction of the process for the preparation of an aryloxyphenoxypropionic acid derivative compound of formula I may be an aliphatic bridged bicyclic tertiary amine selected from the group consisting of 1,4-diazabicyclo[2.2.2.]octane (i.e. DABCO), quinuclidine or tropane. The aryl group of the haloarene compound of formula Ar-Y may be 6-chloro-2-quinoxalinyl, the R' group of the phenolpropionic acid derivative compound of formula II may be selected from the group consisting of hydrogen, methyl, ethyl, 2-isopropylideneamino-oxyethyl or (tetrahydro-2-furanyl)methyl, X may be O, and the catalyst in the reaction of the process for the preparation of an aryloxyphenoxypropionic acid derivative compound of formula I may be DABCO.

The aryl group of the haloarene compound of formula Ar-Y may be selected from the group consisting of substituted phenyl, substituted 2-pyridinyl, substituted 2-benzoxazolyl, substituted 2-quinoxalinyl, substituted 2-benzothiazolyl, substituted naphthyl or substituted 2-benzimidazole, wherein the substituents are selected from the group consisting of halogen, aliphatic, halo aliphatic, alkoxy, thioalkyl, cyano or nitro groups, the R' group of the phenolpropionic acid derivative compound of formula II may be selected from the group consisting of hydrogen, linear or branched alkyl, alkynyl, alkoxyalkyl, aralkyl, haloaryl, alkylideneamino-oxyalkyl or heterocyclyl alkyl group, X is selected from NR" or O, R" is selected from the group consisting of hydrogen, linear or branched aliphatic group, the catalyst in the reaction of the process for the preparation of an aryloxyphenoxypropionic acid derivative compound of formula I is a tertiary amine, and the base in the process may be an inorganic base. The aryl group of the haloarene compound of formula Ar-Y may be selected from the group consisting of substituted phenyl, substituted 2-pyridinyl, substituted 2-benzoxazolyl or substituted 2-quinoxalinyl, wherein the substituents are selected from the group consisting of halogen, aliphatic, halo aliphatic, alkoxy, thioalkyl, cyano or nitro groups, the R' group of the phenolpropionic acid derivative compound of formula II may be selected from the group consisting of hydrogen, linear or branched alkyl, alkynyl, alkoxyalkyl, benzyl, halophenyl, alkylideneamino-oxyalkyl or heterocyclic-ether alkyl group, X is selected from NR" or O, R" may be selected from the group consisting of hydrogen, linear or branched alkyl group, the catalyst in the reaction of the process for the preparation of an aryloxyphenoxypropionic acid derivative compound of formula I may be a bridged bicyclic tertiary amine, and the base in the process may be an inorganic base selected from the group consisting of an alkali metal, alkaline earth metal or ammonium carbonate, bicarbonate, phosphate, hydrogen- or dihydrogenphosphate. The aryl group of the haloarene compound of formula Ar-Y may be selected from the group consisting of halogen substituted 2-quinoxalinyl, halogen substituted 2-benzoxazolyl, halogen and/or haloalkyl substituted 2-pyridinyl, or halogen and/or cyano substituted phenyl, the R' group of the phenolpropionic acid derivative compound of formula II may be selected from the group consisting of hydrogen, linear or branched alkyl, alkynyl, alkoxyalkyl, 2-halophenyl, alkylideneamino-oxyalkyl or 2-heterocyclic-ether alkyl, X is selected from NR" or O, R" may be linear or branched alkyl group, the catalyst in the reaction of the process for the preparation of an aryloxyphenoxypropionic acid derivative compound of formula I may be an aliphatic bridged bicyclic tertiary amine, and the base in the process may be an inorganic base selected from the group consisting of an alkali metal or alkaline earth metal carbonate or bicarbonate. The aryl group of the haloarene compound of formula Ar-Y may be selected from the group consisting of 6-chloro-2-quinoxalinyl, 6-chloro-2-benzoxazolyl, 3-chloro-5-(trifluoromethyl)-2-pyridinyl, 5-(trifluoromethyl)-2-pyridinyl, 4-cyano-2-fluorophenyl, 5-chloro-3-fluoro-2-pyridinyl or 2,4-dichlorophenyl, the R' group of the phenolpropionic acid derivative compound of formula II may be selected from the group consisting of hydrogen, butyl, methyl, ethyl, 2-propynyl, ethoxyethyl, methoxyethyl, 2-fluorophenyl, 2-isopropylideneamino-oxyethyl or (tetrahydro-2-furanyl)methyl, X is selected from NR" or O, R" may be selected from the group consisting of methyl, ethyl, isopropyl or propyl, the catalyst in the reaction of the process for the preparation of an aryloxyphenoxypropionic acid derivative compound of formula I may be an aliphatic bridged bicyclic tertiary amine selected from the group consisting of 1,4-diazabicyclo[2.2.2.]octane (i.e. DABCO), quinuclidine or tropane, and the base in the process may be an inorganic base selected from the group consisting of sodium or potassium carbonate or bicarbonate. The aryl group of the haloarene compound of formula Ar-Y may be 6-chloro-2-quinoxalinyl, the R' group of the phenolpropionic acid derivative compound of formula II may be selected from the group consisting of hydrogen, methyl, ethyl, 2-isopropylideneamino-oxyethyl or (tetrahydro-2-furanyl)methyl, X may be O, the catalyst in the reaction of the process for the preparation of an aryloxyphenoxypropionic acid derivative compound of formula I may be DABCO, and the base in the process may be an inorganic base selected from the group consisting of sodium or potassium carbonate.

The aryl group of the haloarene compound of formula Ar-Y may be selected from the group consisting of substituted phenyl, substituted 2-pyridinyl, substituted 2-benzoxazolyl, substituted 2-quinoxalinyl, substituted 2-benzothiazolyl, substituted naphthyl or substituted 2-benzimidazole, wherein the substituents are selected from the group consisting of halogen, aliphatic, halo aliphatic, alkoxy, thioalkyl, cyano or nitro groups, the R' group of the phenolpropionic acid derivative compound of formula II may be selected from the group consisting of hydrogen, linear or branched alkyl, alkynyl, alkoxyalkyl, aralkyl, haloaryl, alkylideneamino-oxyalkyl or heterocyclyl alkyl group, X is selected from NR" or O, R" is selected from the group consisting of hydrogen, linear or branched aliphatic group, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 2.5:1, the catalyst in the reaction is a tertiary amine, its amount may range from 0.01% to 50% by mole of the moles of the haloarene compound of formula Ar-Y, the base in the process may be an inorganic base, and its amount may range from 1 to 5 moles by mole of the haloarene compound of formula Ar-Y. The aryl group of the haloarene compound of formula Ar-Y may be selected from the group consisting of substituted phenyl, substituted 2-pyridinyl, substituted 2-benzoxazolyl or substituted 2-quinoxalinyl, wherein the substituents are selected from the group consisting of halogen, aliphatic, halo aliphatic, alkoxy, thioalkyl, cyano or nitro groups, the R' group of the phenolpropionic acid derivative compound of formula II may be selected from the group consisting of hydrogen, linear or branched alkyl, alkynyl, alkoxyalkyl, benzyl, halophenyl, alkylideneamino-oxyalkyl or heterocyclic-ether alkyl group, X is selected from NR" or O, R" may be selected from the group consisting of hydrogen, linear or branched alkyl group, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 2.5:1, the catalyst in the reaction may be a bridged bicyclic tertiary amine, its amount may range from 0.01% to 50% by mole of the moles of the haloarene compound of formula Ar-Y, the base in the process may be an inorganic base selected from the group consisting of an alkali metal, alkaline earth metal or ammonium carbonate, bicarbonate, phosphate, hydrogen- or dihydrogenphosphate, and its amount may range from 1 to 5 moles by mole of the haloarene compound of formula Ar-Y. The aryl group of the haloarene compound of formula Ar-Y may be selected from the group consisting of halogen substituted 2-quinoxalinyl, halogen substituted 2-benzoxazolyl, halogen and/or haloalkyl substituted 2-pyridinyl, or halogen and/or cyano substituted phenyl, the R' group of the phenolpropionic acid derivative compound of formula II may be selected from the group consisting of hydrogen, linear or branched alkyl, alkynyl, alkoxyalkyl, 2-halophenyl, alkylideneamino-oxyalkyl or 2-heterocyclic-ether alkyl, X is selected from NR" or O, R" may be linear or branched alkyl group, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 2:1, the catalyst in the reaction may be an aliphatic bridged bicyclic tertiary amine, its amount may range from 0.1% to 35% by mole of the moles of the haloarene compound of formula Ar-Y, the base in the process may be an inorganic base selected from the group consisting of an alkali metal or alkaline earth metal carbonate or bicarbonate, and its amount may range from 1 to 3 moles by mole of the haloarene compound of formula Ar-Y. The aryl group of the haloarene compound of formula Ar-Y may be selected from the group consisting of 6-chloro-2-quinoxalinyl, 6-chloro-2-benzoxazolyl, 3-chloro-5-(trifluoromethyl)-2-pyridinyl, 5-(trifluoromethyl)-2-pyridinyl, 4-cyano-2-fluorophenyl, 5-chloro-3-fluoro-2-pyridinyl or 2,4-dichlorophenyl, the R' group of the phenolpropionic acid derivative compound of formula II may be selected from the group consisting of hydrogen, butyl, methyl, ethyl, 2-propynyl, ethoxyethyl, methoxyethyl, 2-fluorophenyl, 2-isopropylideneamino-oxyethyl or (tetrahydro-2-furanyl)methyl, X is selected from NR" or O, R" may be selected from the group consisting of methyl, ethyl, isopropyl or propyl, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 1.5:1, the catalyst in the reaction may be an aliphatic bridged bicyclic tertiary amine selected from the group consisting of 1,4-diazabicyclo[2.2.2.]octane (i.e. DABCO), quinuclidine or tropane, its amount may range from 1% to 30% by mole of the moles of the haloarene compound of formula Ar-Y, the base in the process may be an inorganic base selected from the group consisting of sodium or potassium carbonate or bicarbonate, and its amount may range from 1 to 2 moles by mole of the haloarene compound of formula Ar-Y. The aryl group of the haloarene compound of formula Ar-Y may be 6-chloro-2-quinoxalinyl, the R' group of the phenolpropionic acid derivative compound of formula II may be selected from the group consisting of hydrogen, methyl, ethyl, 2-isopropylideneamino-oxyethyl or (tetrahydro-2-furanyl)methyl, X may be O, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 1.2:1, the catalyst in the reaction may be DABCO, its amount may range from 1% to 30% by mole of the moles of the haloarene compound of formula Ar-Y, the base in the process may be an inorganic base selected from the group consisting of sodium or potassium carbonate, and its amount may range from 1 to 2 moles by mole of the haloarene compound of formula Ar-Y.

The aryl group of the haloarene compound of formula Ar-Y may be selected from the group consisting of substituted phenyl, substituted 2-pyridinyl, substituted 2-benzoxazolyl, substituted 2-quinoxalinyl, substituted 2-benzothiazolyl, substituted naphthyl or substituted 2-benzimidazole, wherein the substituents are selected from the group consisting of halogen, aliphatic, halo aliphatic, alkoxy, thioalkyl, cyano or nitro groups, the R' group of the phenolpropionic acid derivative compound of formula II may be selected from the group consisting of hydrogen, linear or branched alkyl, alkynyl, alkoxyalkyl, aralkyl, haloaryl, alkylideneamino-oxyalkyl or heterocyclyl alkyl group, X is selected from NR" or O, R" is selected from the group consisting of hydrogen, linear or branched aliphatic group, the catalyst in the reaction of the process for the preparation of an aryloxyphenoxypropionic acid derivative compound of formula I is a tertiary amine, the base in the process may be an inorganic base, and the nonpolar solvent in the reaction of the process may be a hydrocarbon solvent which optionally contains halogen atoms. The aryl group of the haloarene compound of formula Ar-Y may be selected from the group consisting of substituted phenyl, substituted 2-pyridinyl, substituted 2-benzoxazolyl or substituted 2-quinoxalinyl, wherein the substituents are selected from the group consisting of halogen, aliphatic, halo aliphatic, alkoxy, thioalkyl, cyano or nitro groups, the R' group of the phenolpropionic acid derivative compound of formula II may be selected from the group consisting of hydrogen, linear or branched alkyl, alkynyl, alkoxyalkyl, benzyl, halophenyl, alkylideneamino-oxyalkyl or heterocyclic-ether alkyl group, X is selected from NR" or O, R" may be selected from the group consisting of hydrogen, linear or branched alkyl group, the catalyst in the reaction of the process for the preparation of an aryloxyphenoxypropionic acid derivative compound of formula I may be a bridged bicyclic tertiary amine, the base in the process may be an inorganic base selected from the group consisting of an alkali metal, alkaline earth metal or ammonium carbonate, bicarbonate, phosphate, hydrogen- or dihydrogenphosphate, and the nonpolar solvent in the reaction of the process may be a hydrocarbon solvent which optionally contains halogen atoms. The aryl group of the haloarene compound of formula Ar-Y may be selected from the group consisting of halogen substituted 2-quinoxalinyl, halogen substituted 2-benzoxazolyl, halogen and/or haloalkyl substituted 2-pyridinyl, or halogen and/or cyano substituted phenyl, the R' group of the phenolpropionic acid derivative compound of formula II may be selected from the group consisting of hydrogen, linear or branched alkyl, alkynyl, alkoxyalkyl, 2-halophenyl, alkylideneamino-oxyalkyl or 2-heterocyclic-ether alkyl, X is selected from NR" or O, R" may be linear or branched alkyl group, the catalyst in the reaction of the process for the preparation of an aryloxyphenoxypropionic acid derivative compound of formula I may be an aliphatic bridged bicyclic tertiary amine, the base in the process may be an inorganic base selected from the group consisting of an alkali metal or alkaline earth metal carbonate or bicarbonate, and the nonpolar solvent in the reaction of the process may be an aromatic hydrocarbon solvent which optionally contains halogen atoms. The aryl group of the haloarene compound of formula Ar-Y may be selected from the group consisting of 6-chloro-2-quinoxalinyl, 6-chloro-2-benzoxazolyl, 3-chloro-5-(trifluoromethyl)-2-pyridinyl, 5-(trifluoromethyl)-2-pyridinyl, 4-cyano-2-fluorophenyl, 5-chloro-3-fluoro-2-pyridinyl or 2,4-dichlorophenyl, the R' group of the phenolpropionic acid derivative compound of formula II may be selected from the group consisting of hydrogen, butyl, methyl, ethyl, 2-propynyl, ethoxyethyl, methoxyethyl, 2-fluorophenyl, 2-isopropylideneamino-oxyethyl or (tetrahydro-2-furanyl)methyl, X is selected from NR" or O, R" may be selected from the group consisting of methyl, ethyl, isopropyl or propyl, the catalyst in the reaction of the process for the preparation of an aryloxyphenoxypropionic acid derivative compound of formula I may be an aliphatic bridged bicyclic tertiary amine selected from the group consisting of 1,4-diazabicyclo[2.2.2.]octane (i.e. DABCO), quinuclidine or tropane, the base in the process may be an inorganic base selected from the group consisting of sodium or potassium carbonate or bicarbonate, and the nonpolar solvent in the reaction of the process may be an aromatic hydrocarbon solvent which optionally contains halogen atoms. The aryl group of the haloarene compound of formula Ar-Y may be 6-chloro-2-quinoxalinyl, the R' group of the phenolpropionic acid derivative compound of formula II may be selected from the group consisting of hydrogen, methyl, ethyl, 2-isopropylideneamino-oxyethyl or (tetrahydro-2-furanyl)methyl, X may be O, the catalyst in the reaction of the process for the preparation of an aryloxyphenoxypropionic acid derivative compound of formula I may be DABCO, the base in the process may be an inorganic base selected from the group consisting of sodium or potassium carbonate, and the nonpolar solvent in the reaction of the process may be an aromatic hydrocarbon solvent which optionally contains halogen atoms and selected from the group consisting of toluene, benzene, ethylbenzene, xylene, cumene, cymene, mesitylene, biphenyl, chlorobenzene or bromobenzene.

The aryl group of the haloarene compound of formula Ar-Y may be selected from the group consisting of substituted phenyl, substituted 2-pyridinyl, substituted 2-benzoxazolyl, substituted 2-quinoxalinyl, substituted 2-benzothiazolyl, substituted naphthyl or substituted 2-benzimidazole, wherein the substituents are selected from the group consisting of halogen, aliphatic, halo aliphatic, alkoxy, thioalkyl, cyano or nitro groups, the R' group of the phenolpropionic acid derivative compound of formula II may be selected from the group consisting of hydrogen, linear or branched alkyl, alkynyl, alkoxyalkyl, aralkyl, haloaryl, alkylideneamino-oxyalkyl or heterocyclyl alkyl group, X is selected from NR" or O, R" is selected from the group consisting of hydrogen, linear or branched aliphatic group, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 2.5:1, the catalyst in the reaction is a tertiary amine, its amount may range from 0.01% to 50% by mole of the moles of the haloarene compound of formula Ar-Y, the base in the process may be an inorganic base, its amount may range from 1 to 5 moles by mole of the haloarene compound of formula Ar-Y, and the nonpolar solvent in the reaction of the process may be a hydrocarbon solvent which optionally contains halogen atoms. The aryl group of the haloarene compound of formula Ar-Y may be selected from the group consisting of substituted phenyl, substituted 2-pyridinyl, substituted 2-benzoxazolyl or substituted 2-quinoxalinyl, wherein the substituents are selected from the group consisting of halogen, aliphatic, halo aliphatic, alkoxy, thioalkyl, cyano or nitro groups, the R' group of the phenolpropionic acid derivative compound of formula II may be selected from the group consisting of hydrogen, linear or branched alkyl, alkynyl, alkoxyalkyl, benzyl, halophenyl, alkylideneamino-oxyalkyl or heterocyclic-ether alkyl group, X is selected from NR" or O, R" may be selected from the group consisting of hydrogen, linear or branched alkyl group, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 2.5:1, the catalyst in the reaction may be a bridged bicyclic tertiary amine, its amount may range from 0.01% to 50% by mole of the moles of the haloarene compound of formula Ar-Y, the base in the process may be an inorganic base selected from the group consisting of an alkali metal, alkaline earth metal or ammonium carbonate, bicarbonate, phosphate, hydrogen- or dihydrogenphosphate, its amount may range from 1 to 5 moles by mole of the haloarene compound of formula Ar-Y, and the nonpolar solvent in the reaction of the process may be a hydrocarbon solvent which optionally contains halogen atoms. The aryl group of the haloarene compound of formula Ar-Y may be selected from the group consisting of halogen substituted 2-quinoxalinyl, halogen substituted 2-benzoxazolyl, halogen and/or haloalkyl substituted 2-pyridinyl, or halogen and/or cyano substituted phenyl, the R' group of the phenolpropionic acid derivative compound of formula II may be selected from the group consisting of hydrogen, linear or branched alkyl, alkynyl, alkoxyalkyl, 2-halophenyl, alkylideneamino-oxyalkyl or 2-heterocyclic-ether alkyl, X is selected from NR" or O, R" may be linear or branched alkyl group, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 2:1, the catalyst in the reaction may be an aliphatic bridged bicyclic tertiary amine, its amount may range from 0.1% to 35% by mole of the moles of the haloarene compound of formula Ar-Y, the base in the process may be an inorganic base selected from the group consisting of an alkali metal or alkaline earth metal carbonate or bicarbonate, its amount may range from 1 to 3 moles by mole of the haloarene compound of formula Ar-Y, and the nonpolar solvent in the reaction of the process may be an aromatic hydrocarbon solvent which optionally contains halogen atoms. The aryl group of the haloarene compound of formula Ar-Y may be selected from the group consisting of 6-chloro-2-quinoxalinyl, 6-chloro-2-benzoxazolyl, 3-chloro-5-(trifluoromethyl)-2-pyridinyl, 5-(trifluoromethyl)-2-pyridinyl, 4-cyano-2-fluorophenyl, 5-chloro-3-fluoro-2-pyridinyl or 2,4-dichlorophenyl, the R' group of the phenolpropionic acid derivative compound of formula II may be selected from the group consisting of hydrogen, butyl, methyl, ethyl, 2-propynyl, ethoxyethyl, methoxyethyl, 2-fluorophenyl, 2-isopropylideneamino-oxyethyl or (tetrahydro-2-furanyl)methyl, X is selected from NR" or O, R" may be selected from the group consisting of methyl, ethyl, isopropyl or propyl, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 1.5:1, the catalyst in the reaction may be an aliphatic bridged bicyclic tertiary amine selected from the group consisting of 1,4-diazabicyclo[2.2.2.]octane (i.e. DABCO), quinuclidine or tropane, its amount may range from 1% to 30% by mole of the moles of the haloarene compound of formula Ar-Y, the base in the process may be an inorganic base selected from the group consisting of sodium or potassium carbonate or bicarbonate, its amount may range from 1 to 2 moles by mole of the haloarene compound of formula Ar-Y, and the nonpolar solvent in the reaction of the process may be an aromatic hydrocarbon solvent which optionally contains halogen atoms. The aryl group of the haloarene compound of formula Ar-Y may be 6-chloro-2-quinoxalinyl, the R' group of the phenolpropionic acid derivative compound of formula II may be selected from the group consisting of hydrogen, methyl, ethyl, 2-isopropylideneamino-oxyethyl or (tetrahydro-2-furanyl)methyl, X may be O, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 1.2:1, the catalyst in the reaction may be DABCO, its amount may range from 1% to 30% by mole of the moles of the haloarene compound of formula Ar-Y, the base in the process may be an inorganic base selected from the group consisting of sodium or potassium carbonate, its amount may range from 1 to 2 moles by mole of the haloarene compound of formula Ar-Y, and the nonpolar solvent in the reaction of the process may be an aromatic hydrocarbon solvent which optionally contains halogen atoms and selected from the group consisting of toluene, benzene, ethylbenzene, xylene, cumene, cymene, mesitylene, biphenyl, chlorobenzene or bromobenzene.

The aryl group of the haloarene compound of formula Ar-Y may be selected from the group consisting of substituted phenyl, substituted 2-pyridinyl, substituted 2-benzoxazolyl, substituted 2-quinoxalinyl, substituted 2-benzothiazolyl, substituted naphthyl or substituted 2-benzimidazole, wherein the substituents are selected from the group consisting of halogen, aliphatic, halo aliphatic, alkoxy, thioalkyl, cyano or nitro groups, the R' group of the phenolpropionic acid derivative compound of formula II may be selected from the group consisting of hydrogen, linear or branched alkyl, alkynyl, alkoxyalkyl, aralkyl, haloaryl, alkylideneamino-oxyalkyl or heterocyclyl alkyl group, X is selected from NR" or O, R" is selected from the group consisting of hydrogen, linear or branched aliphatic group, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 2.5:1, the catalyst in the reaction is a tertiary amine, its amount may range from 0.01% to 50% by mole of the moles of the haloarene compound of formula Ar-Y, the base in the process may be an inorganic base, its amount may range from 1 to 5 moles by mole of the haloarene compound of formula Ar-Y, the nonpolar solvent in the reaction of the process may be a hydrocarbon solvent which optionally contains halogen atoms, and the optionally additional polar solvent in the reaction of the process may be a polar aprotic solvent. The aryl group of the haloarene compound of formula Ar-Y may be selected from the group consisting of substituted phenyl, substituted 2-pyridinyl, substituted 2-benzoxazolyl or substituted 2-quinoxalinyl, wherein the substituents are selected from the group consisting of halogen, aliphatic, halo aliphatic, alkoxy, thioalkyl, cyano or nitro groups, the R' group of the phenolpropionic acid derivative compound of formula II may be selected from the group consisting of hydrogen, linear or branched alkyl, alkynyl, alkoxyalkyl, benzyl, halophenyl, alkylideneamino-oxyalkyl or heterocyclic-ether alkyl group, X is selected from NR" or O, R" may be selected from the group consisting of hydrogen, linear or branched alkyl group, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 2.5:1, the catalyst in the reaction may be a bridged bicyclic tertiary amine, its amount may range from 0.01% to 50% by mole of the moles of the haloarene compound of formula Ar-Y, the base in the process may be an inorganic base selected from the group consisting of an alkali metal, alkaline earth metal or ammonium carbonate, bicarbonate, phosphate, hydrogen- or dihydrogenphosphate, its amount may range from 1 to 5 moles by mole of the haloarene compound of formula Ar-Y, the nonpolar solvent in the reaction of the process may be a hydrocarbon solvent which optionally contains halogen atoms, and the optionally additional polar solvent in the reaction of the process may be a polar aprotic solvent. The aryl group of the haloarene compound of formula Ar-Y may be selected from the group consisting of halogen substituted 2-quinoxalinyl, halogen substituted 2-benzoxazolyl, halogen and/or haloalkyl substituted 2-pyridinyl, or halogen and/or cyano substituted phenyl, the R' group of the phenolpropionic acid derivative compound of formula II may be selected from the group consisting of hydrogen, linear or branched alkyl, alkynyl, alkoxyalkyl, 2-halophenyl, alkylideneamino-oxyalkyl or 2-heterocyclic-ether alkyl, X is selected from NR" or O, R" may be linear or branched alkyl group, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 2:1, the catalyst in the reaction may be an aliphatic bridged bicyclic tertiary amine, its amount may range from 0.1% to 35% by mole of the moles of the haloarene compound of formula Ar-Y, the base in the process may be an inorganic base selected from the group consisting of an alkali metal or alkaline earth metal carbonate or bicarbonate, its amount may range from 1 to 3 moles by mole of the haloarene compound of formula Ar-Y, the nonpolar solvent in the reaction of the process may be an aromatic hydrocarbon solvent which optionally contains halogen atoms, and the optionally additional polar solvent in the reaction of the process may be a polar aprotic solvent selected from the group consisting of ketones, nitriles, alkyl lactams, lactones, sulfoxides, sulfones, nitro compounds, urea derivatives or amides. The aryl group of the haloarene compound of formula Ar-Y may be selected from the group consisting of 6-chloro-2-quinoxalinyl, 6-chloro-2-benzoxazolyl, 3-chloro-5-(trifluoromethyl)-2-pyridinyl, 5-(trifluoromethyl)-2-pyridinyl, 4-cyano-2-fluorophenyl, 5-chloro-3-fluoro-2-pyridinyl or 2,4-dichlorophenyl, the R' group of the phenolpropionic acid derivative compound of formula II may be selected from the group consisting of hydrogen, butyl, methyl, ethyl, 2-propynyl, ethoxyethyl, methoxyethyl, 2-fluorophenyl, 2-isopropylideneamino-oxyethyl or (tetrahydro-2-furanyl)methyl, X is selected from NR" or O, R" may be selected from the group consisting of methyl, ethyl, isopropyl or propyl, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 1.5:1, the catalyst in the reaction may be an aliphatic bridged bicyclic tertiary amine selected from the group consisting of 1,4-diazabicyclo[2.2.2.]octane (i.e. DABCO), quinuclidine or tropane, its amount may range from 1% to 30% by mole of the moles of the haloarene compound of formula Ar-Y, the base in the process may be an inorganic base selected from the group consisting of sodium or potassium carbonate or bicarbonate, its amount may range from 1 to 2 moles by mole of the haloarene compound of formula Ar-Y, the nonpolar solvent in the reaction of the process may be an aromatic hydrocarbon solvent which optionally contains halogen atoms, and the optionally additional polar solvent in the reaction of the process may be a polar aprotic solvent selected from the group consisting of ketones, nitriles, alkyl lactams, lactones, sulfoxides, sulfones, nitro compounds, urea derivatives or amides. The aryl group of the haloarene compound of formula Ar-Y may be 6-chloro-2-quinoxalinyl, the R' group of the phenolpropionic acid derivative compound of formula II may be selected from the group consisting of hydrogen, methyl, ethyl, 2-isopropylideneamino-oxyethyl or (tetrahydro-2-furanyl)methyl, X may be O, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 1.2:1, the catalyst in the reaction may be DABCO, its amount may range from 1% to 30% by mole of the moles of the haloarene compound of formula Ar-Y, the base in the process may be an inorganic base selected from the group consisting of sodium or potassium carbonate, its amount may range from 1 to 2 moles by mole of the haloarene compound of formula Ar-Y, the nonpolar solvent in the reaction of the process may be an aromatic hydrocarbon solvent which optionally contains halogen atoms and selected from the group consisting of toluene, benzene, ethylbenzene, xylene, cumene, cymene, mesitylene, biphenyl, chlorobenzene or bromobenzene, and the optionally additional polar solvent in the reaction of the process may be a polar aprotic solvent selected from the group consisting of acetone, methyl isobutyl ketone, methyl ethyl ketone, benzonitrile, acetonitrile, N-methyl-2-pyrrolidone, butyrolactone, dimethyl formamide, dimethyl acetamide, dimethyl sulfoxide, sulfolane, nitromethane, nitrobenzene, tetramethyl urea, 1,3-dimethyl-2-imidazolidinone or N-N'-dimethylpropylene urea.

The aryl group of the haloarene compound of formula Ar-Y may be selected from the group consisting of substituted phenyl, substituted 2-pyridinyl, substituted 2-benzoxazolyl, substituted 2-quinoxalinyl, substituted 2-benzothiazolyl, substituted naphthyl or substituted 2-benzimidazole, wherein the substituents are selected from the group consisting of halogen, aliphatic, halo aliphatic, alkoxy, thioalkyl, cyano or nitro groups, the R' group of the phenolpropionic acid derivative compound of formula II may be selected from the group consisting of hydrogen, linear or branched alkyl, alkynyl, alkoxyalkyl, aralkyl, haloaryl, alkylideneamino-oxyalkyl or heterocyclyl alkyl group, X is selected from NR" or O, R" is selected from the group consisting of hydrogen, linear or branched aliphatic group, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 2.5:1, the catalyst in the reaction is a tertiary amine, its amount may range from 0.01% to 50% by mole of the moles of the haloarene compound of formula Ar-Y, the base in the process may be an inorganic base, its amount may range from 1 to 5 moles by mole of the haloarene compound of formula Ar-Y, the nonpolar solvent in the reaction of the process may be a hydrocarbon solvent which optionally contains halogen atoms, the optionally additional polar solvent in the reaction of the process may be a polar aprotic solvent, and its amount may range from 0.001% to 10% by weight of the weight of the nonpolar solvent. The aryl group of the haloarene compound of formula Ar-Y may be selected from the group consisting of substituted phenyl, substituted 2-pyridinyl, substituted 2-benzoxazolyl or substituted 2-quinoxalinyl, wherein the substituents are selected from the group consisting of halogen, aliphatic, halo aliphatic, alkoxy, thioalkyl, cyano or nitro groups, the R' group of the phenolpropionic acid derivative compound of formula II may be selected from the group consisting of hydrogen, linear or branched alkyl, alkynyl, alkoxyalkyl, benzyl, halophenyl, alkylideneamino-oxyalkyl or heterocyclic-ether alkyl group, X is selected from NR" or O, R" may be selected from the group consisting of hydrogen, linear or branched alkyl group, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 2.5:1, the catalyst in the reaction may be a bridged bicyclic tertiary amine, its amount may range from 0.01% to 50% by mole of the moles of the haloarene compound of formula Ar-Y, the base in the process may be an inorganic base selected from the group consisting of an alkali metal, alkaline earth metal or ammonium carbonate, bicarbonate, phosphate, hydrogen- or dihydrogenphosphate, its amount may range from 1 to 5 moles by mole of the haloarene compound of formula Ar-Y, the nonpolar solvent in the reaction of the process may be a hydrocarbon solvent which optionally contains halogen atoms, the optionally additional polar solvent in the reaction of the process may be a polar aprotic solvent, and its amount may range from 0.001% to 10% by weight of the weight of the nonpolar solvent. The aryl group of the haloarene compound of formula Ar-Y may be selected from the group consisting of halogen substituted 2-quinoxalinyl, halogen substituted 2-benzoxazolyl, halogen and/or haloalkyl substituted 2-pyridinyl, or halogen and/or cyano substituted phenyl, the R' group of the phenolpropionic acid derivative compound of formula II may be selected from the group consisting of hydrogen, linear or branched alkyl, alkynyl, alkoxyalkyl, 2-halophenyl, alkylideneamino-oxyalkyl or 2-heterocyclic-ether alkyl, X is selected from NR" or O, R" may be linear or branched alkyl group, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 2:1, the catalyst in the reaction may be an aliphatic bridged bicyclic tertiary amine, its amount may range from 0.1% to 35% by mole of the moles of the haloarene compound of formula Ar-Y, the base in the process may be an inorganic base selected from the group consisting of an alkali metal or alkaline earth metal carbonate or bicarbonate, its amount may range from 1 to 3 moles by mole of the haloarene compound of formula Ar-Y, the nonpolar solvent in the reaction of the process may be an aromatic hydrocarbon solvent which optionally contains halogen atoms, the optionally additional polar solvent in the reaction of the process may be a polar aprotic solvent selected from the group consisting of ketones, nitriles, alkyl lactams, lactones, sulfoxides, sulfones, nitro compounds, urea derivatives or amides, and its amount may range from 0.01% to 5% by weight of the weight of the nonpolar solvent. The aryl group of the haloarene compound of formula Ar-Y may be selected from the group consisting of 6-chloro-2-quinoxalinyl, 6-chloro-2-benzoxazolyl, 3-chloro-5-(trifluoromethyl)-2-pyridinyl, 5-(trifluoromethyl)-2-pyridinyl, 4-cyano-2-fluorophenyl, 5-chloro-3-fluoro-2-pyridinyl or 2,4-dichlorophenyl, the R' group of the phenolpropionic acid derivative compound of formula II may be selected from the group consisting of hydrogen, butyl, methyl, ethyl, 2-propynyl, ethoxyethyl, methoxyethyl, 2-fluorophenyl, 2-isopropylideneamino-oxyethyl or (tetrahydro-2-furanyl)methyl, X is selected from NR" or O, R" may be selected from the group consisting of methyl, ethyl, isopropyl or propyl, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 1.5:1, the catalyst in the reaction may be an aliphatic bridged bicyclic tertiary amine selected from the group consisting of 1,4-diazabicyclo[2.2.2.]octane (i.e. DABCO), quinuclidine or tropane, its amount may range from 1% to 30% by mole of the moles of the haloarene compound of formula Ar-Y, the base in the process may be an inorganic base selected from the group consisting of sodium or potassium carbonate or bicarbonate, its amount may range from 1 to 2 moles by mole of the haloarene compound of formula Ar-Y, the nonpolar solvent in the reaction of the process may be an aromatic hydrocarbon solvent which optionally contains halogen atoms, the optionally additional polar solvent in the reaction of the process may be a polar aprotic solvent selected from the group consisting of ketones, nitriles, alkyl lactams, lactones, sulfoxides, sulfones, nitro compounds, urea derivatives or amides, and its amount may range from 0.01% to 5% by weight of the weight of the nonpolar solvent. The aryl group of the haloarene compound of formula Ar-Y may be 6-chloro-2-quinoxalinyl, the R' group of the phenolpropionic acid derivative compound of formula II may be selected from the group consisting of hydrogen, methyl, ethyl, 2-isopropylideneamino-oxyethyl or (tetrahydro-2-furanyl)methyl, X may be O, the molar ratio of the phenolpropionic acid derivative compound of formula II versus the haloarene compound of formula Ar-Y in the reaction of the process for the preparation of an aryloxyphenoxypropionic acid derivative compound of formula I may range from 0.9:1 to 1.2:1, the catalyst in the reaction may be DABCO, its amount may range from 1% to 30% by mole of the moles of the haloarene compound of formula Ar-Y, the base in the process may be an inorganic base selected from the group consisting of sodium or potassium carbonate, its amount may range from 1 to 2 moles by mole of the haloarene compound of formula Ar-Y, the nonpolar solvent in the reaction of the process may be an aromatic hydrocarbon solvent which optionally contains halogen atoms and selected from the group consisting of toluene, benzene, ethylbenzene, xylene, cumene, cymene, mesitylene, biphenyl, chlorobenzene or bromobenzene, the optionally additional polar solvent in the reaction of the process may be a polar aprotic solvent selected from the group consisting of acetone, methyl isobutyl ketone, methyl ethyl ketone, benzonitrile, acetonitrile, N-methyl-2-pyrrolidone, butyrolactone, dimethyl formamide, dimethyl acetamide, dimethyl sulfoxide, sulfolane, nitromethane, nitrobenzene, tetramethyl urea, 1,3-dimethyl-2-imidazolidinone or N-N'-dimethylpropylene urea, and its amount may range from 0.1% to 2% by weight of the weight of the nonpolar solvent.

The following examples are included for illustrative purposes only and should not be construed as limitations on the invention claimed herein.

### Examples

### Comparative example 1: Propaquizafop synthesis with TBAHS (Tetrabutylammonium hydrogen sulphate)

190 mLToluene are charged into 500 mL round bottom flask, followed by 63g (R)-2-(4-hydroxy-phenoxy)-propionic acid 2-isopropylideneaminooxy-ethyl ester as 75% toluene solution, 32g 2,6-dichloro-quinoxaline, 2g of TBAHS and 28g potassium carbonate.

The mixture is heated to 90°C and the water produced during the condensation is continuously removed by azeotropic distillation under reduced pressure. After 5 hours, the reaction completion is checked for 2,6-dichloro-quinoxaline disappearance. 200 ml water are added for inorganic salts dissolution, then the organic phase is washed once again to neutral. The toluene is distilled off under reduced pressure to get technical 71.5 g of technical product (yield 86.3%) and 70% R-isomer content.

### Comparative example 2: Propaquizafop synthesis with TBAHS

190 mL Toluene are charged into 500 mL round bottom flask, followed by 63g (R)-2-(4-hydroxy-phenoxy)-propionic acid 2-isopropylideneaminooxy-ethyl ester as 75% toluene solution, 32g 2,6-dichloro-quinoxaline, 2g of TBAHS and 28g potassium carbonate.

The mixture is heated to 90°C and the water produced during the condensation is continuously removed by azeotropic distillation under reduced pressure. After 17 hours, the reaction is completed, 200 ml water are added for inorganic salts dissolution, then the organic phase is washed once again to neutral. The toluene is distilled off under reduced pressure to get 67 g of technical product (yield 84.3%) and R-isomer content 92%.

### Example 1: Propaquizafop synthesis with DABCO

170 ml Toluene are charged into 500 mL round bottom flask, followed by 66g (R)-2-(4-hydroxy-phenoxy)-propionic acid 2-isopropylideneaminooxy-ethyl ester as 75% toluene solution, 32g 2,6-dichloro-quinoxaline, 2.7g of 1.4 Diazabicyclo[2,2,2]octane (DABCO) and 28g potassium carbonate.

The mixture is heated to 90°C and the water produced during the condensation is continuously removed by azeotropic distillation under reduced pressure. After 5 hours, the reaction completion is checked for 2,6-dichloro-quinoxaline disappearance and found complete. 280 ml water are added for inorganic salts dissolution, then the organic phase is washed once again to neutral. The toluene is distilled off under reduced pressure to get 72 g of technical Propaquizafop (yield 93.5%) and R-isomer content more than 99%.

### Example 2: Propaquizafop synthesis with DABCO

170 ml Toluene are charged into 500 mL round bottom flask, followed by 64g (R)-2-(4-hydroxy-phenoxy)-propionic acid 2-isopropylideneaminooxy-ethyl ester as 73% toluene solution, 32g 2,6-dichloro-quinoxaline, 2.7g of 1.4 Diazabicyclo[2,2,2]octane (DABCO) and 28g potassium carbonate.

The mixture is heated to 90°C and the water produced during the condensation is continuously removed by azeotropic distillation under reduced pressure. After 5 hours, the reaction completion was checked for 2,6-dichloro-quinoxaline disappearance and found complete. 280 ml water are added for inorganic salts dissolution, then the organic phase is washed once again to neutral. The toluene is distilled off under reduced pressure to get 71 g of technical Propaquizafop (yield 94.5%) and R-isomer content 99.6%.

By the process of this invention, propaquizafop compound of formula I is produced in an excellent yield without any deterioration of the final product optical purity.

### Example 3: Quizalofop-ethyl synthesis with DABCO

170 ml Toluene are charged into 500 mL round bottom flask, followed by 49.3g Ethyl 2-(4-hydroxyphenoxy)propionate as 72% toluene solution, 32g 2,6-dichloro-quinoxaline, 2.7g of 1.4 Diazabicyclo[2,2,2]octane (DABCO) and 28g potassium carbonate.

The mixture is heated to 90°C and the water produced during the condensation is continuously removed by azeotropic distillation under reduced pressure. After 5 hours, the reaction completion was checked for 2,6-dichloro-quinoxaline disappearance and found complete. 280 ml water are added for inorganic salts dissolution, then the organic phase is washed once again to neutral. The toluene is distilled off under reduced pressure to get 59 g of technical Quizalofop-ethyl (yield 92%) and R-isomer content 99%.

### Comparative table of experiments

The following examples are carried out according to the procedure disclosed in the previous examples, but with different reaction conditions and reactant quantities. The obtained results are shown in table 1.

**Table 1**

| **Catalyst (Ratio catalyst vs Ar-Y mol:mol)** | **Ratio K₂CO₃ vs Ar-Y mol:mol** | **Temperature of reaction (°C)** | **Ratio compound of formula II vs Ar-Y mol:mol** | **Reaction time, (h)** | **Yield, %** | **% R-isomer content** |
|---|---|---|---|---|---|---|
| TBAHS (0.037) | 1.27 | 90 | 1.15 | 8 | 85.2 | 68 |
| TBAHS (0.037) | 1.27 | 85 | 0.9 | 6 | 78.6 | 98 |
| TBAHS (0.037) | 1.27 | 90 | 1.2 | 10 | 86.0 | 70 |
| TBAHS (0.037) | 1.5 | 90 | 1.15 | 4 | 84.8 | 81 |
| TBAHS (0.074) | 1.27 | 80 | 1.15 | 12 | 85.4 | 63 |
| | | | | | | |
| DABCO (0.15) | 1.27 | 90 | 1.05 | 6 | 90.8 | 99 |
| DABCO (0.15) | 1.27 | 90 | 1.15 | 5 | 89.1 | 99 |
| DABCO (0.15) | 1.27 | 90 | 1.15 | 5 | 91.1 | 99 |
| DABCO (0.2) | 1.27 | 90 | 1.15 | 5 | 90.0 | 99 |
| DABCO (0.3) | 1.27 | 90 | 1.15 | 4 | 91.3 | 99 |

Reactions conditions: Ar-Y: 2,6-dichloro-quinoxaline; Compound of formula II: (R)-2-(4-hydroxy-phenoxy)-propionic acid 2-isopropylideneaminooxy-ethyl ester; Azeotropic distillation of water at temperature of the reaction; solvent: toluene; DABCO: 1,4-diazabicyclo[2.2.2.]octane; TBAHS: Tetrabutylammonium hydrogen sulphate.

Table 1 shows that when the catalyst is the tertiary amine 1,4-diazabicyclo[2.2.2.]octane, the experiments are reproduceable, and the yield and content of R-isomer are increased compared with the quaternary ammonium TBAHS of the prior art.

## Claims

1. A process for the preparation of an aryloxyphenoxypropionic acid derivative compound of formula I 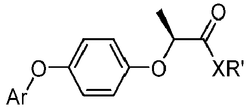
wherein Ar is an aryl group, R' is selected from the group consisting of hydrogen, linear or branched aliphatic, alkoxyalkyl, alicyclic, aralkyl, haloaryl, alkylideneamino-oxyalkyl or heterocyclyl alkyl group, X is selected from NR" or O, and R" is selected from the group consisting of hydrogen, linear or branched aliphatic group, which comprises the reaction in a nonpolar solvent of a phenolpropionic acid derivative compound of formula II 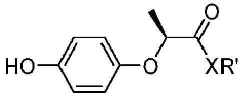
with a haloarene compound of formula Ar-Y when Y is a halogen atom, in the presence of an inorganic or organic base and a catalyst selected from the group of tertiary amines.

2. The process according to claim 1, wherein the water formed as byproduct is removed from the reaction.

3. The process according to any of previous claims, wherein the temperature of the reaction is in the range from 60 to 200 ºC.

4. The process according to any of previous claims, wherein the pressure of the reaction is normal atmospheric pressure or reduced pressure.

5. The process according to any of previous claims, wherein the nonpolar solvent is a hydrocarbon solvent which optionally contains halogen atoms.

6. The process according to any of previous claims, wherein the inorganic base is selected from the group consisting of an alkali metal, alkaline earth metal or ammonium carbonate, bicarbonate, phosphate, hydrogen- or dihydrogenphosphates.

7. The process according to any of previous claims, wherein the amount of the inorganic or organic base ranges from 1 to 5 moles by mole of the haloarene compound of formula Ar-Y.

8. The process according to any of previous claims, wherein the catalyst is a bridged bicyclic tertiary amine.

9. The process according to claim 8, wherein the bridged bicyclic tertiary amine is an aliphatic bridged bicyclic tertiary amine.

10. The process according to claim 9, wherein the aliphatic bridged bicyclic tertiary amine is selected from the group consisting of 1,4-diazabicyclo[2.2.2.]octane, quinuclidine or tropane.

11. The process according to any of previous claims, wherein the amount of catalyst ranges from 0.01% to 50% by mole of the moles of the haloarene compound of formula Ar-Y.

12. The process according to any of previous claims, wherein the aryl group Ar is selected from the group consisting of substituted phenyl, substituted 2-pyridinyl, substituted 2-benzoxazolyl, substituted 2-quinoxalinyl, substituted 2-benzothiazolyl, substituted naphthyl or substituted 2-benzimidazole, wherein the substituents are selected from the group consisting of halogen, aliphatic, halo aliphatic, alkoxy, thioalkyl, cyano or nitro groups.

13. The process according to any of previous claims, wherein the R' group is selected from the group consisting of hydrogen, linear or branched alkyl, alkynyl, alkoxyalkyl, aralkyl, haloaryl, alkylideneamino-oxyalkyl or heterocyclyl alkyl group.

14. The process according to any of previous claims, wherein X is selected from NR" or O, and R" is selected from the group consisting of hydrogen, linear or branched alkyl group.

15. The process according to any of previous claims, wherein molar ratio of the phenolpropionic acid derivative compound of formula II versus the haloarene compound of formula Ar-Y ranges from 0.9:1 to 2.5:1.
